(19) Europäisches Patentamt
European Patent Office
Office européen des brevets

(11) **EP 1 830 292 A1**

(12) **EUROPEAN PATENT APPLICATION**

(43) Date of publication:
**05.09.2007 Bulletin 2007/36**

(51) Int Cl.:
*G06F 19/00* (2006.01)

(21) Application number: **07004338.5**

(22) Date of filing: **02.03.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK YU**<br><br>(30) Priority: **03.03.2006 EP 06075506**<br>**01.09.2006 EP 06018383**<br><br>(71) Applicant: **Mentis Cura ehf.**<br>**101 Reykjavik (IS)** | (72) Inventors:<br>• **Gudmundsson, Steinn**<br>**113 Reykjavik (IS)**<br>• **Johannesson, Gisli Holmar**<br>**210 Gardabaer (IS)**<br>• **Helgason, Johannes**<br>**300 Akranes (IS)**<br>• **Johnsen, Kristinn**<br>**101 Reykjavik (IS)**<br><br>(74) Representative: **Plougmann & Vingtoft A/S**<br>**Sundkrogsgade 9,**<br>**P.O. Box 831**<br>**2100 Copenhagen Ø (DK)** |

(54) **Method of constructing and using a reference tool to generate a discriminatory signal for indicating a medical condition of a subject**

(57) This invention relates to constructing a reference tool and using the reference tool for distinguishing between a medical condition of a subject and a reference subject. This tool may be considered as a reference "map" consisting of one or several numbers of reference groups, where the subjects within the same groups share one or more common characteristics, e.g. age, sex, medical condition, etc. The present invention therefore relates to seeing whether a subject to be diagnosed falls within one or more of said groups simply by comparing processed biological data collected from the subject with the reference "map".

Fig. 1

**Description**

FIELD OF THE INVENTION

The present Invention relates to a reference tool to be used as reference for distinguishing between a medical condition of a subject and a reference subject. The present invention further relates to a method and a system for generating a discriminatory signal for indicating a medical condition of a subject.

BACKGROUND OF THE INVENTION

Today, it is extremely difficult if not impossible to diagnose whether a subject suffers from e.g. a neurological disease. One way of measuring the neurological condition of the brain is to perform biological measurement, e.g. by implementing an electroencephalography (EEG) technique. This technique measures the electrical activity of the brain as varying spontaneous potentials (SPs) over time through a plurality of electrodes placed at different locations on the scalp. It is, however, extremely difficult to diagnose a patient based on the resulting neurological data (showing the SPs as a function of time) due to the enormous information comprised therein. It is extremely difficult or even possible to distinguish between e.g. a healthy subject and a subject suffering from a medical condition.
In recent years, several methods have been developed to diagnose whether a subject suffer from a particular disease. Most of these methods are based on supervised or unsupervised classification. Examples of these references are:

Jerebko A K et al: "Multiple Neural Network Classification Scheme for Detection of Colonic Polyps in CT Conolography Data Sets"; Academic Radiology, Reston, VA, US, vol. 10, no. 2, Feb. 2003, p. 154-160, XP009049614 ISSN: 1076-6332;
Christodoulou C I et al: "Medical diagnostic system using ensembles of neural SOFM classifiers" Electronics, Circuits and Systems, 1999, Proceedings of ICECS '99 the 6th IEEE international conference on Pafos, Cyprus 5-8 Sept. 1999, Piscataway, NJ, USA, IEEE, US, vol. 1, 5, Sept 1999, p. 121-124, XP010361455, ISBN: 0-7803-5682-9;
Gunter S et al: "An evaluation of ensembles methods in handwritten word recognition based on feature selection" Pattern recognition, 2004, ICPR 2004. Proceedings of the 17th international conference in Cambridge, UK, AUG 23-26, Piscataway, NJ, USA, IEEE, vol. 1, 23. August 2004, p. 388-392, XP010724266, ISBN: 0-7695-2128-2:
Jain A K et al: "Statistical Pattern Recognition: A review", IEEE Transaction on Pattern Analysis and Machine Intelligence, IEEE Service Center, Los Alamitos, CA, US, vol. 22, No. 1m January 2000, p. 4-37, XP000936788 ISSN: 0162-8828.

These methods relate to supervised or unsupervised classification of data, i.e. how a computer based on input data can distinguish a pattern in the data. Although said references show that that false-positive rates are reduced when diagnosing a person, they are far from being advanced enough to diagnose a subject at a very early stage with high reliability.
There is therefore a need for a highly advanced reference tool which, when implemented, is capable of diagnosing a person at a very early stage, and which is even capable of making more than one diagnose at the same time.

BRIEF DESCRIPTION OF THE INVENTION

The object of the present invention is to construct a reference tool, and a method of using the reference tool to distinguish between a medical condition of a subject and a reference subject. This tool may be considered as a reference "map" consisting of one or more reference groups where the subjects within the same groups share one or more common characteristics, e.g. age, sex, medical condition, etc. The object of the present invention is therefore to see whether a subject to be diagnosed falls within one or more of said groups simply by comparing processed biological data collected from the subject with the reference "map".
Furthermore, the object of the present invention is to provide a corresponding apparatus for constructing said reference tool and an apparatus for implementing the reference tool for generating a discriminatory signal.
According to one aspect, the present Invention relates to a method of constructing a reference tool based on biosignal data collected from one or more groups of reference subjects, wherein each group represents reference subjects having at least one common characteristic, the method comprising:

- defining one or more reference features representing a common characteristic of the references subjects within the same group,
- determining reference feature values for said reference features for each respective reference subject,

- defining a feature property domain comprising domain elements where each element is defined by one of said reference features or a combination of two or more of said reference features,
- determining a posterior probability vector for each respective reference subject based on said feature property domain, wherein each respective element of the posterior probability vector refers to said domain elements and indicates the probability that a reference subject of a particular group belongs to said group in terms of said domain elements,
- applying a filtering process on said posterior probability vectors, said filtering process being based on removing those vectors or vector elements that are above or below a pre-defined threshold value, the remaining vectors or vector elements being implemented for constructing a reference distribution for said reference subjects as a function of said domain elements.

By defining the reference features, only relevant parts of the biosignal data are selected, i.e. those parts where there is a correlation in the data for the subjects within the same group. The result in that only the relevant information from the biosignal data are processed which is a great advantage since often the information contains a huge amount data that are not relevant. As an example, Electroencephalography data (EEG) contain an enormous amount of information that is very difficult to process. According to the present invention, said groups contain reference subjects that share one or more common characteristics, and the data (in this example the EEG data) are to be used as reference data. The advantage of defining said reference features is that only the portion of the data which is considered to be relevant for constructing the reference tool and which is common to the reference subjects within the same group is used.

Furthermore, by applying said filtering process of the posterior probability vectors it is possible to separate said groups from each other so that the only vector elements having certain or sufficient high probability values are used as preferred data for constructing the reference tool. The result is a reference tool consisting of one or more group domains where each domain reflects the characteristic of each respective group very well. The result is a highly advanced reference tool consisting of at least one group or a reference group of reference subjects that share said one or more common characteristics. Accordingly, by comparing analogous posterior probability vectors of a subject that is to be diagnosed, it is possible to tell whether this subject belongs to the one or more groups or not, and the subject can be diagnosed at an early stage. If e.g. a subject belongs to two or more groups, that could be an indicator that the subject suffers from two or more medical conditions.

According to the present Invention the term subject means a human being, but the term can just as well relate to animals and other biological organisms.

In one embodiment, the reference features are selected from a group consisting of the absolute delta power, the absolute theta power, the absolute alpha power, the absolute beta power, the absolute gamma power, the relative delta power, the relative theta power, the relative alpha power, the relative beta power, the relative gamma power, the total power, the peak frequency, the median frequency, the spectral entropy, the DFA scaling exponent (alpha band oscillations), the DFA scaling exponent (beta band oscillations) and the total entropy.

In one embodiment, the domain elements of said feature property domain comprise non-repetitive combinations of two or more of said reference features.

In one embodiment, the step of determining the posterior probability vector is based on a calculation method selected from a group consisting of: k-NN nearest neighbor scheme (k-NN), support vector machines (SVM), linear discriminant analysis, quadratic discriminant analysis, regularized discriminant analysis, Logistic regression, naive Bayes classifier, hidden Markov model classifiers, neural network based classifiers including multi-layer perceptron networks and radial basis function networks, support vector machines (SVM), Least-squares SVM, Classification Tree-based classifiers including Classification and Regression Trees (CART), ID3, C4.5, C5.0, AdaBoost, and ArcX4, Tree-based ensemble classifier including Random Forests™.

In one embodiment, the biosignal data within the same groups are collected during similar activity of the reference subjects within the same group. In that way, very consistent data are obtained. An example of such an activity to collect e.g. EEG data from the reference subject while the reference subject has his eyes closed, or to collect data while the subjects are observing a particular Image, or solving a particular problem, etc.

In one embodiment, wherein said characteristics of the reference subjects are selected from the following characteristics:

- the gender of the subject,
- the habit-related actions performed by the subjects,
- the medical condition of the subjects,
- the particular neurological disease of the subjects,
- the specific handedness of the subjects,
- the age of the subjects,
- whether the subjects are healthy or not,

- the physical condition of the subjects.

According to another aspect, the present invention relates to a computer program product for instructing a processing unit to execute the method step of any of the above mentioned method steps when the product is run on a computer.

According to yet another aspect, the present invention relates to an apparatus for constructing a reference tool based on biosignal data collected from one or more groups of reference subjects, wherein each group represents reference subjects having at least one common characteristic, the apparatus comprising:

- a processor for pre-scanning the biosignal data and, based thereon, defining one or more reference features representing a common characteristic of the reference subjects within the same group,
- a processor for determining reference feature values for said reference features for each respective reference subject,
- means for defining a feature property domain comprising domain elements where each element is defined by one of said reference features or a combination of two or more of said reference features,
- a processor for determining a posterior probability vector for each respective reference subject based on said feature property domain, wherein each respective element of the posterior probability vector refers to said domain elements and indicates the probability that a reference subject of a particular group belongs to said group In terms of said domain elements,
- a processor for applying a filtering process on said posterior probability vectors, said filtering process being based on removing those vectors or vector elements that are above or below a pre-defined threshold value, the remaining vectors or vector elements being implemented for constructing a reference distribution for said reference subjects as a function of said domain elements.

In one embodiment, the apparatus further comprises a receiver adapted to receive biosignal data from said reference subjects.

According to yet another aspect, the present invention relates to a method of using a pre-stored reference tool for generating a discriminatory signal for indicating a medical condition of a subject based on biosignal data collected from the subject, the reference tool being constructed based on biosignal data collected from one or more groups of reference subjects, each group representing reference subjects having at least one common characteristic, said reference tool being constructed by means of:

defining one or more reference features representing a common characteristic of the references subjects within the same group,
determining reference feature values for said reference features for each respective reference subject,
defining a feature property domain comprising domain elements where each element is defined by one of said reference features or a combination of two or more of said reference features,
determining a posterior probability vector for each respective reference subject based on said feature property domain, wherein each respective element of the posterior probability vector refers to said domain elements and indicates the probability that a reference subject of a particular group belongs to said group in terms of said domain elements,
applying a filtering process on said posterior probability vectors, said filtering process being based on removing those vectors or vector elements that are above or below a pre-defined threshold value, the remaining vectors or vector elements being implemented for constructing a reference distribution for said reference subjects as a function of said domain elements, where the method comprises:

- determining analogous feature values for the subject,
- determining an analogous posterior probability vector for said subject,
- evaluating whether said posterior probability vector for said subject lies within said distribution for said reference subjects.

As mentioned above, by implementing such a highly advantageous reference tool, it is possible to diagnose a subject at an early stage by determining said analogous posterior probability vector and comparing it with said reference distribution.

According to the present invention the term discriminatory signal means the result of said comparison, i.e. a signal indicating whether said subject belongs totally or partly within/outside one or more of said groups. This can be based on an evaluation performed by a physician or on an automatic evaluation. Accordingly, the subject can be diagnosed positive/negative, partly positive/negative based on the discriminatory signal.

In one embodiment, the features are selected from a group consisting of the absolute delta power, the absolute theta power, the absolute alpha power, the absolute beta power, the absolute gamma power, the relative delta power, the relative theta power, the relative alpha power, the relative beta power, the relative gamma power, the total power, the peak frequency, the median frequency, the spectral entropy, the DFA scaling exponent (alpha band oscillations), the DFA scaling exponent (beta band oscillations) and the total entropy.

In one embodiment, said one or more biosignal data comprise electroencephalographic (EEG) data.

In one embodiment, the biosignal data comprise data resulting from biosignal measurements selected from the group consisting of:

- magnetic resonance imaging (MRI),
- functional magnetic resonance imaging (FMRI),
- magneto-encephalographic (MEG) measurements,
- positron emission tomography (PET),
- CAT scanning (Computed Axial Tomography) and
- single photon emission computerized tomography (SPECT).

In one embodiment, the medical condition is a neurological condition.

In one embodiment, the neurological condition is selected from the group consisting of Alzheimer's disease, multiple sclerosis, mental conditions including depressive disorders, bipolar disorder and schizophrenic disorders, Parkinson's' disease, epilepsy, migraine, Vascular Dementia (VaD), Fronto-temporal dementia, Lewy bodies dementia, Creutzfeld-Jacob disease and vCJD ("mad cow's disease").

In one embodiment, the groups are defined such that they share at least one known characteristic with the subject. This can e.g. be that the subject and the reference group are the same age, sex, etc.

In one embodiment, the present invention further relates to a computer program product for instructing a processing unit to execute the above method steps of using a pre-stored reference tool for generating a discriminatory signal for Indicating a medical condition of a subject when the product is run on a computer.

According to yet another aspect, the present invention relates to an apparatus for using a pre-stored reference tool for generating a discriminatory signal for indicating a medical condition of a subject based on biosignal data collected from the subject, the reference tool being constructed based on biosignal data collected from one or more groups of reference subjects, each group representing reference subjects having at least one common characteristic, said reference tool being constructed by means of:

defining one or more reference features representing a common characteristic for the references subjects within the same group,

determining reference feature values for said reference features for each respective reference subject,

defining a feature property domain comprising domain elements where each element is defined by one of said reference features or a combination of two or more of said reference features,

determining a posterior probability vector for each respective reference subject based on said feature property domain, wherein each respective element of the posterior probability vector refers to said domain elements and indicates the probability that a reference subject of a particular group belongs to said group in terms of said domain elements,

applying a filtering process on said posterior probability vectors, said filtering process being based on removing those vectors or vector elements that are above or below a pre-defined threshold value, the remaining vectors or vector elements being implemented for constructing a reference distribution for said reference subjects as a function of said domain elements, wherein the apparatus comprises:

- a processor for determining analogous feature values for the subject,
- a processor for determining an analogous posterior probability vector for said subject,
- a processor for evaluating whether said posterior probability vector for said subject lies within said distribution for said reference subjects.

In an embodiment, the apparatus further comprises a receiver and a transmitter, wherein the receiver is adapted to receive said biosignal data from the subject and the transmitter is adapted to transmit the resulting generated discriminatory signal.

According to yet another embodiment, the present invention relates to the use of a pre-stored reference tool for generating a discriminatory signal for indicating a medical condition of a subject based on biosignal data collected from the subject, the reference tool being constructed based on biosignal data collected from one or more groups of reference subjects, each group representing reference subjects having at least one common characteristic, said reference tool being constructed by means of:

defining one or more reference features representing a common characteristic of the references subjects within the same group,

determining reference feature values for said reference features for each respective reference subject,

defining a feature property domain comprising domain elements where each element is defined by one of said reference features or a combination of two or more of said reference features,

determining a posterior probability vector for each respective reference subject based on said feature property domain, wherein each respective element of the posterior probability vector refers to said domain elements and indicates the probability (18-20) that a reference subject of a particular group belongs to said group in terms of said domain elements,

applying a filtering process on said posterior probability vectors, said filtering process being based on removing those vectors or vector elements that are above or below a pre-defined threshold value, the remaining vectors or vector elements being implemented for constructing a reference distribution for said reference subjects as a function of said domain elements, wherein the use comprises:

- determining analogous feature values for the subject,
- determining an analogous posterior probability vector for said subject,
- evaluating whether said posterior probability vector for said subject lies within said distribution for said reference subjects.

According to yet another aspect, the present invention relates to a method of using a pre-stored reference tool for diagnosing a medical condition of a subject based on biosignal data collected from the subject, the reference tool being constructed based on biosignal data collected from one or more groups of reference subjects, each group representing reference subjects having at least one common characteristic, said reference tool being constructed by means of:

defining one or more reference features representing a common characteristic for the references subjects within the same group,

determining reference feature values for said reference features for each respective reference subject,

defining a feature property domain comprising domain elements where each element is defined by one of said reference features or a combination of two or more of said reference features,

determining a posterior probability vector for each respective reference subject based on said feature property domain, wherein each respective element of the posterior probability vector refers to said domain elements and indicates the probability that a reference subject of a particular group belongs to said group in terms of said domain elements,

applying a filtering process on said posterior probability vectors, said filtering process being based on removing those vectors or vector elements that are above or below a pre-defined threshold value, the remaining vectors or vector elements being implemented for constructing a reference distribution for said reference subjects as a function of said domain elements, wherein the method comprises:

- determining analogous feature values for the subject,
- determining an analogous posterior probability vector for said subject,
- evaluating whether said posterior probability vector for said subject lies within said distribution for said reference subjects.

The aspects of the present invention may each be combined with any of the other aspects. These and other aspects of the invention will be apparent from and elucidated with reference to the embodiments described hereinafter.

BRIEF DESCRIPTION OF THE DRAWINGS

Embodiments of the invention will be described, by way of example only, with reference to the drawings, in which

Figure 1 depicts graphically a construction of a reference tool according to the present invention based on biosignal data collected from two groups of reference subjects,

Figures 2-4 illustrates graphically an example of a distribution of reference subjects for the first property from Fig. 1,

Figure 5 shows how the probability distribution based on Fig. 2-4 for a single property could be,

Figure 6 illustrates graphically a separation between two groups, group A and B, with respect to a single property,

Figure 7 illustrates graphically a reference tool according to the present invention showing the distribution for three groups, group A, B and C, as a function of properties from the posterior probability vectors,

Figure 8 depicts graphically a possible scenario of implementing the reference tool,

Figure 9 shows a method of constructing a reference tool adapted for providing a reference for generating a discriminatory signal for indicating a medical condition of a subject,

Figs. 10-12 illustrate schematically a possible distribution for these properties for all the reference subjects in groups A and B,

Figure 13 shows a reference tool after filtering out the vectors or vector elements that do not fulfill the filtering criterion ,

Figure 14a-c illustrates graphically an example of defining and calculating features from biosignal data, e.g. EEG data,

Figure 15 shows an apparatus according to the present Invention for constructing said reference tool,

Figure 16 shows a flow diagram of method steps for using said reference tool to diagnose a subject,

Figure 17 shows an apparatus according to the present invention for using said pre-stored reference tool for generating a discriminatory signal for indicating a medical condition of a subject,

Figure 18 illustrates a comparison between the classification performances for two sets evaluated using the 3-NN scheme,

Figure 19 illustrates the same comparison, but using the SVM classification scheme to obtain the feature pair classifiers,

Figure 20 shows a distribution for one property based on experimental results, where for each property the posterior probability is calculated using a SVM classifier,

Figure 21 illustrates the posterior probabilities for two subjects, an Alzheimer's subject, circles, and a control subject, and

Figure 22 shows experiment results for a particular group where the posterior probability is plotted as a function of the properties, and

Figure 23 and 24 illustrate the resulting effect when administering the probe compound scopolamine, where Fig. 23 shows the features stem from measurements before administration of the scopolamine, and Fig. 24 demonstrates the response of the same features by considering the ratio of their values pre- and post-administration.

DESCRIPTION OF EMBODIMENTS

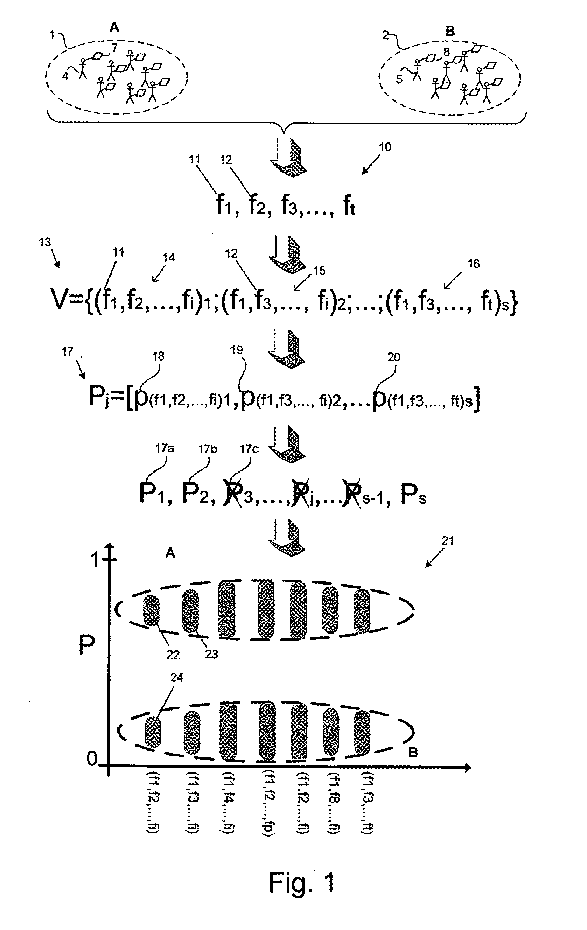

Figure 1 depicts graphically a construction of a reference tool according to the present invention based on biosignal data collected from two groups 1, 2 of reference subjects 4, 5, where the reference subjects within the same group share at least one common characteristic, such as the same age, sex, particular neurological disease, specific handedness, etc.

Reference features "f1" 11, "f2" 12, "f3" 10 that represent the common characteristics are initially defined based on biosignal data 7, 8 collected from the reference subjects within the two groups 1, 2. As an example, reference feature f1 11 can relate to the absolute delta power, f2 12 can relate to the absolute theta power, etc. As shown here, the index t refers to the number of features from the two groups 1, 2. The definition of the reference features is typically made by initially pre-screening the biological data within each respective group and in that way detecting a correlation in the data set. As an example, a common characteristic within group (A) 1 may include the absolute delta power since it is remarkably high (or low) and that the spectral entropy is remarkably low (or high), whereas a common characteristic within group (B) 2 may be that the DFA scaling exponent (alpha band oscillations) is remarkably high. Based on the above, reference features for each respective group are defined. As an example, reference features for group (A) 1 may be f1-f3 and for group (B) f4-f6. Subsequently, the feature values are calculated, e.g. the absolute delta power, the spectral entropy, alpha band oscillations, etc.

A feature property domain V 13 that is common for all the groups must now be defined, wherein the domain 13 comprises domain elements 14-16 defined based on said reference features 11, 12, 10. The domain elements are referred to as properties and may consist of a single reference feature or a combination of said reference features. Typically, such a feature domain may be defined manually by a user (e.g. a technician, a doctor, a clinician, etc.). In one embodiment, the domain $V$ 13 consists of a non repetitive combination of two or more reference features f1-f6. If it is assumed that the index N represents a combination parameter which indicates how many features are to be combined. For t=3, domain $V$ 13 becomes:

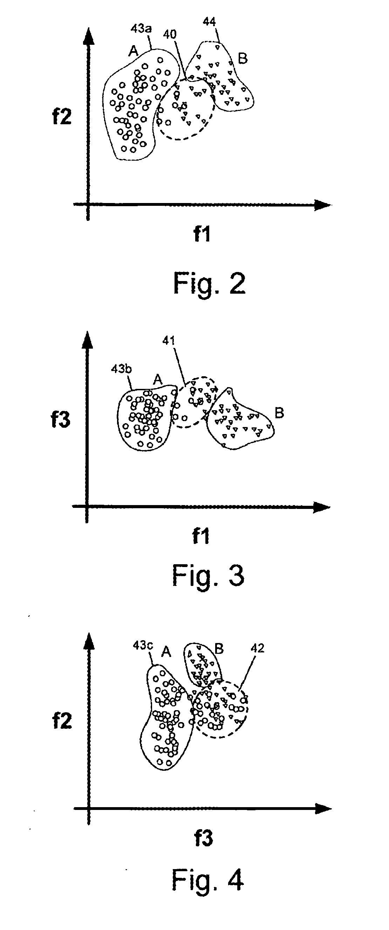

V={(f1,f2,f3);(f1,f2,f4);(f1,f2,f5);(f1,f2,f6);(f1,f3,f4);(f1,f3,f5);(f1,f3,f6);(f1,f4,f5);(f1,f4,f6); (f1,f5,f6)},

where the number of properties is ten. Each domain element is referred to as a property, i.e. (f1,f2,f3) 14 is a first property, (f1,f2,f4) 15 is a second property, etc. For each respective property a statistical distribution for the reference subject within groups (A)-(b) 1-2 is evaluated. The three dimensional first property is illustrated graphically in Figs. 2-4 for the first property (f1,f2,f3), where in Fig. 2 feature values f1 and f2 are indicated for all the reference subjects within groups (A)-(B) 1-2, Fig. 3 feature values f1 and f3 are plotted, and in Fig. 4 feature values f2 and f3 are plotted. The subjects within group (A) are marked with circles and the subjects within group (B) 2 are marked with triangles. As shown here, part of the distributions are separated from each other (e.g. areas 43-44 in Fig. 2), whereas parts of the distributions overlap 40-41. Since both the distributions form a kind of "cluster" they indicate that features f1-f3 are features that characterize both the subjects in group A and group B. However, these two groups could be distinguished from each other by the fact that In group A the subjects are males, but in group B they are females.

A posterior probability vector Pj 17 is now calculated. Such calculations have been reported in the literature, e.g. by Duda et al. "Pattern Classification", John Wiley & Sons, Inc. (2001) p. 61 and by Qing Tao, Gao-Wei Wu, Fei-Yue Wang, Jue Wang, Key Lab. of Complex Syst. & Intelligence Sci., Chinese Acad, of Sci., Beijing, China, Neural Networks, IEEE Transactions on Publication Date: Nov. 2005, Volume: 16, Issue: 6, p. 1561- 1573, ISSN: 1045-9227, INSPEC Accession Number: 8658863, Digital Object Identifier: 10.1109/TNN.2005.857955 Posted online: 2005-11-07 09:51:44.0, which are hereby incorporated by reference. The vector elements 18-20 of the vector 17 give the probability of whether subject j belonging to a particular group in terms of a particular property actually belongs to this group. This means that considering the property (f1,f2,f3), the vector element 18 p(f1,f2,...,fi)1 gives the probability that the a subject within a given group belongs to this group in terms of property (f1,f2,f3). Based on the above where the features are given by f1-f6, the vector would be given by:

$$P_{subject\ j,(A,B,C)} = [p_{(f1,12,13)};\ p_{(f1,f2,f4)};\ p_{(f1,f2,f5)};\ p_{(f1,f2,f6)};\ p_{(f1,f3,f4)};\ p_{(f1,f3,f5)};\ p_{(f1,f3,f6)};\ p_{(f1,f4,f5)};\ p_{(f1,f4,f6)};\ p_{(f1,f5,f6)}].$$

Here it is assumed that the properties comprise non-repetitive combinations of the features 11, 12. This is, however, not necessarily the case.

Example of posterior probability estimation

Consider a classification problem between two classes A and B with respect to an arbitrary set of features X. To solve the classification problem a classifier seeks to define a surface, S, in X space that best separates A and B, with respect to some selected measure. Selecting a specific distance measure one finds the minimal signed distance, d, to S. The elements of A and B form distributions in d space that describe the posterior probabilities for belonging to class A or B. The posterior probabilities can be estimated using a number of methods. The most commonly used technique is maximum likelihood estimation. Let P(D|d) denote the posterior probability that an element with distance d belongs to class D. Then P(A|d)+P(B|d) = 1. Consider a training set (di,yi) where di denotes the distance of element i and yi = 1 if the element belongs to class A and yi = -1 if it belongs to class B. Let ti = (yi+1)/2 and pi = P(A|di). In order to estimate the posterior probability from a given set of data one can parameterize the distribution. One way to do this is to assume that pi = 1/(1+exp(a di + b)). The parameters a and b are then found by minimizing the log likelihood of the training data :
Min -Σi (ti log(pi)+(1-ti)log(1- pi)).

According to Figs. 2-4 if a subject belonging to e.g. group A lies within areas 43a-c, the probability value that this particular subject belongs to group A in terms of this property is high, whereas if a subject belonging to group A lies within area 43a in Fig. 2, but within the overlapped areas 41-42 in Figs. 3-4, the probability is lower. Accordingly, the first element of the posterior probability vector, p(f1,f2,f3)1 18 for this particular subject would in the former case be higher than in the latter case.

The first element, $P_{(f1,f2,f3)}$ 18, gives the posterior probability value that a subject within a particular group with respect to.property (f1,f2,f3) actually belongs to this group based on the distribution shown in Figs. 2-4.

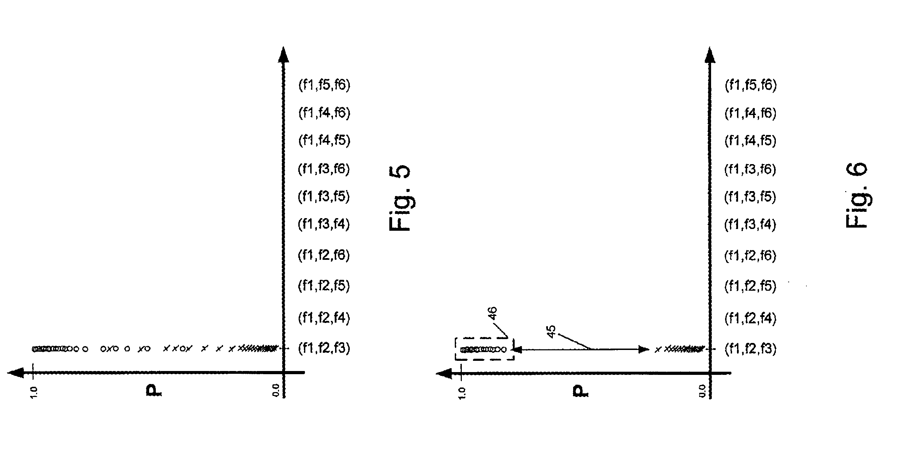

Figure 5 depicts graphically how the probability distribution based on Fig. 2-4 could be. The horizontal axis represents the properties and the vertical axis the distribution. Due to the overlap 40-42 in Figs. 2-4, there is no separation in the distribution in Fig. 5. Here it has been assumed that when calculating the posterior probability vectors group B has been used as a reference group. Therefore, low P values for group B indicate a high likelihood that the subjects belong to group B. The calculation of the posterior probability vector will be discussed in more details below. By considering this one-dimensional distribution (note that this is only the first element in the posterior probability vector) due to the overlap it would be difficult to identify whether a subject belongs to group A or B in relation to property (f1,f2,f3).

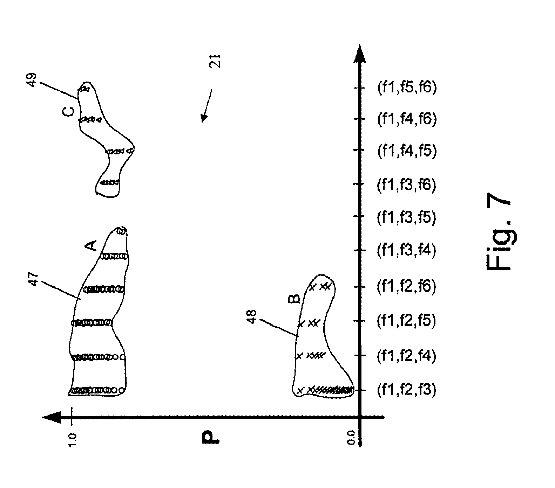

Referring now to Fig. 1, by filtering out those vectors or vector elements that do not have a sufficiently high (or low in the case of group B) probability value one can create a separation 45 between the groups A and B with respect to property (f1,f2,f3). This is illustrated graphically in Fig. 6. In this way, if e.g. the reference subjects have Alzheimer's, the reliability that a test subject having a p(f1,f2,f3) value which is within area 46 may be interpreted to be high based on this single property (f1,f2,f3). It follows that the reliability that the reference subjects within group A actually have the particular characteristic can be controlled. This follows of course in that a very good reference for this one particular property is provided.

The vector elements for all the subsequent domain elements 14-16 (p(f1,f2,f4), p(f1,f2,f5) etc.) are now calculated. In order to make said separation for the subsequent vector elements, a threshold value must be provided, and based on the threshold value all posterior vectors (as whole) or vector elements are removed for all the properties. An example of providing a threshold value is to select all vector elements that have a value of 0.8 or higher and delete all subsequent elements (vectors). Fig. 7 illustrates graphically a reference tool 21 according to the present invention for three groups, A, B and C 47-49 as a function of said properties. Here, a distribution 49 for a possible third group 49 having different characteristics is also plotted in Fig. 7. The number of groups can of course be much larger than shown here, and also the number of reference features. It is obvious from the above that if the posterior vectors (or vector elements) that did not fulfill the threshold value had not been deleted, the distributions in Fig. 6 would (or could) overlap. Therefore, it would be difficult or even impossible to determine whether a test subject belongs to a certain group or not.

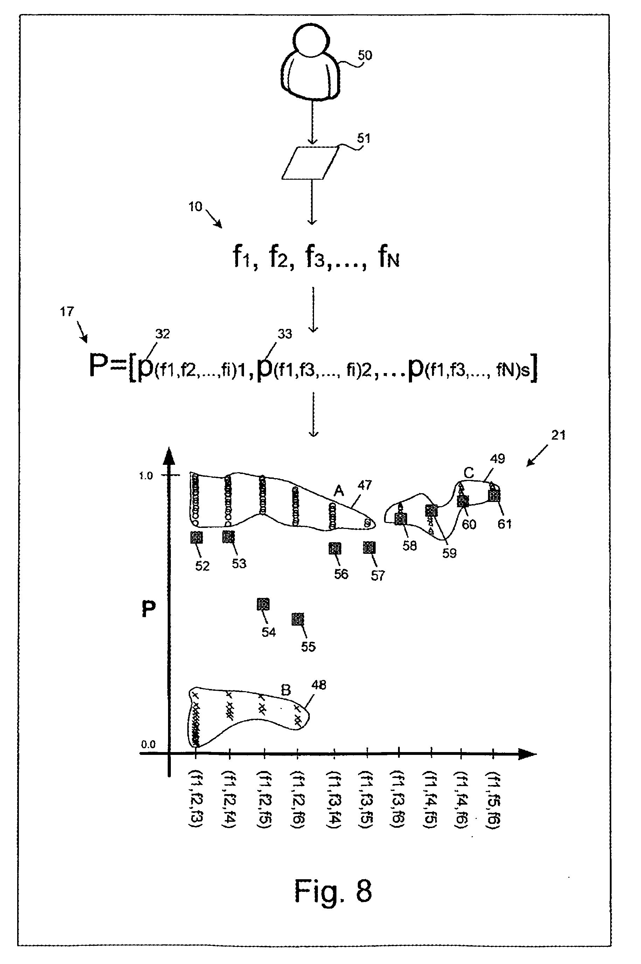

Accordingly, Fig. 7 is implemented as a reference tool 21 for generating a discriminatory signal for indicating a medical condition of a test subject, such as a neurological condition. One implementation of such reference tool as shown in Fig. 8 is that equivalent biosignal data 51 are collected from a test subject 50. Based on the received data, feature values are calculated and associated to said features 10. As an example, if the reference features comprises the spectral entropy, the total entropy, DFA scaling exponent etc., the feature values would be the spectral entropy value, the total entropy value, DFA scaling exponent value for this test subject. Based on the example above, this would accordingly correspond to determining the feature values associated to the "f1"-"f6" features. A corresponding posterior probability vector for the test subject is then determined, and the vector is then compared to the reference tool in fig. 7, i.e. to the reference distribution.

Fig. 8 depicts a possible scenario where the first six elements 52-57 in the posterior probability vector neither belong neither to reference group A 47 nor reference group B 48, whereas the last four elements 58-61 belong to group C 49. If, as an example, reference group C is a group that suffers from a particular neurological disease, this could indicate that the test subject 50 suffers from this disease.

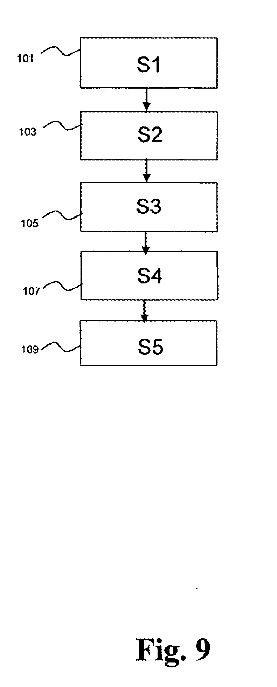

Figure 9 shows a method of constructing a reference tool according to the present invention for providing a reference for generating a discriminatory signal for indicating a medical condition of a subject. Initially one or more groups of subjects to be used as reference groups are selected (S1) 101 based on at least one selection criteria. The selection of the group of the reference subject is typically based on the characteristics of the group. All members of each respective group have a particular characteristic, e.g. a particular neurological disease, specific handedness, same age and sex, are healthy subjects of various age groups, etc. Many other characteristics may be relevant. The groups may be defined such that they fit the known characteristics of a subject to be classified, e.g. same age and handedness if the goal is to determine whether it has a particular neurological condition. Then the variable characteristic between the groups are utilized for classification of the neurological condition.

In a subsequent step (52) 103, biosignal data are obtained based on biosignal measurements obtained from a biosignal measuring device adapted for placement on said reference subjects. These biosignal data can be obtained in various ways, e.g. by the subject or by other technical or medical experts. As an example, the data could be collected from the subject at home by using the necessary measuring equipment. One or more biosignal measurements comprise blosignal data selected e.g. from the group consisting of electroencephalography (EEG) magnetic resonance imaging (MRI), functional magnetic resonance imaging (FMRI), magneto-encephalography (MEG) measurements, positron emission tomography (PET), CAT scanning (Computed Axial Tomography) and single photon emission computerized tomography (SPECT). The biosignal measurement can also be related to other physiological parameters, e.g. gene expression levels in blood or other tissues found e.g. by micro-arrays or PCR, concentration of specific proteins and enzymes in e.g. blood and urine samples, basic physiological parameters such as temperature, sex, age, ethnic origin, weight, height and so forth. Furthermore, the bioslgnal data may be of environmental or historic origin, e.g. main occupation, disease history, living conditions, diet, details of drug and alcohol use, smoking and so forth. It is implied that the data is computerized, e.g. that images are digitized, etc.

In another embodiment, the step of obtaining the data from the subjects comprises obtaining the data during similar activity as performed by the reference subjects when obtaining the reference data. As an example, if the data from the reference subjects were obtained by EEG while they had their eyes closed, it is preferred that the data obtained from the patient is obtained while he/she has his/her eyes closed, or if the data obtained from the reference persons were obtained while observing an image or a text, a similar activity should be performed by the patient when obtaining the data.

In a subsequent step (S3) 105 reference features are calculated for the biosignal data, wherein these features represent common characteristics for the reference subjects within the same group. As an example, all members of each respective group may have a particular characteristic, e.g. a particular neurological disease, specific handedness, the same age and sex. Many other characteristics may be relevant. The reference subjects may be classified according to existing medical records and by thorough examination by medical doctors or other specialists who determine whether each subject belongs to the target groups, e.g. groups with particular neurological conditions such as dementia of the Alzheimer's type. In one embodiment, such biosignal data are collected during controlled clinical trials where each subject undergoes thorough medical examination by specialist medical doctors or other types of skilled persons, e.g. skilled technicians, who determine whether trial candidates fulfill the prescribed definition for each group. The groups are defined so that they fit the known characteristics of a subject to be classified, e.g. same age and handedness if the goal is to determine whether it has a particular neurological condition. Then the variable characteristics between the groups are used for classification of the neurological condition. This will be discussed in more detail below. The reference features may be implemented as single reference features or as reference feature sets consisting of a combination of two or more of said features. The selection of the features is preferably made by "pre-scanning" the data from each of the reference persons and checking which features are suitable for using as reference features. A basic condition that needs to be fulfilled when defining the reference features is that there is a correlation between the features in the data for preferably all the test persons. An example of such reference features is when the biosignal originates from EEG, e.g. the absolute delta power, the absolute theta power, the relative theta power, the spectral frequency, the total power, the DFA scaling exponent (alpha band oscillations), etc. A more exhaustive list of such reference features is given in the description. Accordingly, based on the type of disease some reference features may be more suitable than other reference features. As an example, for a disease A it might be preferred to use the absolute delta power, the absolute theta power or the relative theta power due to the high correlation in these features between the test subjects, whereas for a disease B it might be preferred to use relative theta power, spectral frequency, total power, DFA scaling exponent (alpha band oscillations) as features. Subsequently, the feature values that are associated with the defined features are calculated. In the above mentioned examples, this corresponds to calculating the delta power, the absolute theta power, the relative theta power values, or power, spectral frequency, total power, DFA scaling exponent (alpha band oscillations) values. These calculated values are then used to evaluate the distribution of the feature values as discussed previously in Figs. 2-4. One example of a model is a statistical distribution, e.g. a Gaussian distribution, of the calculated feature values for the reference persons, or the 3-D distribution shown in Figs. 2-4.

Another embodiment takes all features into account, but determines the weight the features should have in order to maximally separate the groups under consideration. Let $f_i, i \in \{1,2,...,N\}$ denote the set of features calculated from the bio-signal data, and Nf be the number of features considered. Consider two balanced groups, A and B, in the sense that the number of subjects in each group is roughly equal. Let Nf denote the total number of subjects in the groups. In practice it is not feasible to consider all the features at once while solving a classification problem since the main danger is over-fitting the data. Let N denote the number of features considered at once. A rule of thumb is not to consider more than $N \leq \dfrac{N_s}{10}$. Let $V_i \in \{f_{i1}, f_{is},...,f_{iN}\}$ be the feature property domain indicating the set of all non-repetitive combinations of features, these combinations being termed properties. Here, $f_{i1}$ corresponds to $(f1,f2,...,fi)_1)$ 14 in Fig. 1, $f_{i1}$ corresponds to $(f1,f3,...,fi)_2)$, etc. As an example, consider $f \in \{1,2,3\}$ and N=2 then V={(1,2),(1,3),(2,3)}.

In a subsequent step (S4) 107, for each respective reference subject assigned to a given group a posterior probability vector is calculated. Such a calculation requires that for each element i in V a classifier is found which is then used to estimate said posterior probabilities for each subject with respect to one of the groups, e.g. the probability that subject j belongs to group A, based on the distribution of the features in Vi (see Figs. 2-4). The classifier is a multidimensional pattern recognition method, e.g. a k-NN nearest neighbor scheme (k-NN), a support vector machine (SVM), a linear discriminant analysis, a quadratic discriminant analysis, a regularized discriminant analysis, a Logistic regression, a naive Bayes classifier, hidden Markov model classifiers, a neural network based classifiers including multi-layer perceptron networks and radial basis function networks, support vector machines (SVM), Least-squares SVM, Classification Tree-based classifiers including Classification and Regression Trees (CART), ID3, C4.5, C5.0, AdaBoost, and ArcX4, Tree-based ensemble classifier including Random Forests™. The chosen classifier is then used to construct a boundary In feature space that best separates the groups according to the criteria the particular classifier is based on. The posterior probability vector is determined from the boundary found, usually as function of distance from the boundary and from the estimated distribution of the training set, e.g. the data from the groups, parameterized as a function of the distance from the said boundary. These probabilities are denoted Pij. Consider an ideal property k in the sense that Pij=1 for all subjects j belonging to group A and Pij=0 for all subjects j belonging to group B meaning that there is no overlap in the distribution for the two groups for a given property (e.g. said (f1,f2,f3) property shown In Figs. 2-4). That would indicate that the classifier correctly classifies all subjects in the training set and, provided that over-fitting has not occurred that it is a good predictor. It turns out that such an ideal distribution of Pij for a particular property is a distribution that maximizes the variance of Pij. A principal component analysis (PCA) identifies independent combinations of variables, i.e. uncorrelated, which maximize the variance. In praxis PCA is performed by examining the eigenvectors and corresponding eigenvalues of the normalized correlation matrix. These eigenvectors are termed pca-eigenvectors. The pca-eigenvector with the largest eigenvalue is the Independent combination of properties that maximizes the variance; the pca-eigenvector with the second largest eigenvalue describes the combination with second largest variance and so forth. In other words, the variance of the combinations described by the pca-eigenvectors of the normalized correlation matrix is proportional to their eigenvalues. Let W be the matrix constructed by the pca-eigenvectors, i.e. the vectors form the columns of W. The pca-posterior probability matrix is defined by Ppca=PW. After this mapping, columns corresponding to pca-eigenvectors with maximum eigenvalues will be as close as possible to the ideal posterior probability distribution with respect to classification. We can consider several such columns and repeat the classification in pca-posterior probability space. In this embodiment the reference feature values calculated are based on all the actual chosen features and are in fact the pca-posterior probabilities. In a worked database one could store the raw data, the features of the reference groups, in order to build the property maps, the pca-posterior probability values for all properties, the matrix W and the corresponding eigenvalues.

When a new subject is to be classified, e.g. a potential patient to be diagnosed, features are calculated from the biosignal obtained. The results are subsequently compared to the data in the database that contains the reference feature values. The procedure is then to determine the posterior probabilities for the new subject in the same way as mentioned previously for each property which results in the posterior probability vector for the subject,

$$P^{subj} = \left( P_1^{subj}, P_2^{subj}, \ldots \right)$$ 17. Then the stored matrix W is used to find the corresponding pca-posterior probabilities, $P_{pca,subj} = P_{subj}W$. Choosing the most relevant components according to the eigenvalues of the matrix W, a new classification is performed with respect to the same components in Ppca obtained from the database. Here only one multidimensional classification is performed using the chosen components of Ppca and comparing them to the training data that consists of the same chosen components from Ppca. This will for example result in a single posterior probability that the new subject belongs to a particular group and is the basis for classification, e.g. prediction that said subject belongs to the particular group. In praxis, decision, classification or diagnosis is made based on a chosen cutoff probability that corresponds to an accepted confidence level. A full diagnosis can involve several such classifications where the subject is compared to several characteristic groups.

In the subsequent step (S5) 109 a filtering process is applied for said features or feature sets. This is done by evaluating said posterior probability vectors with regard to the variance of the probability elements in said vectors, the evaluation resulting in a selection of vectors having variance above/below pre-defined threshold value.

Example 1:

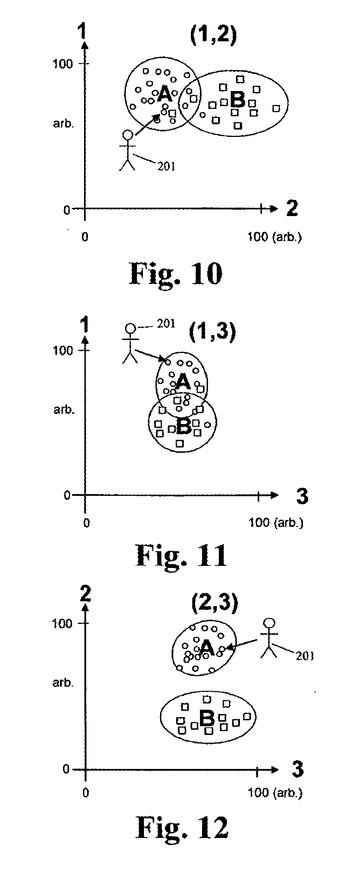

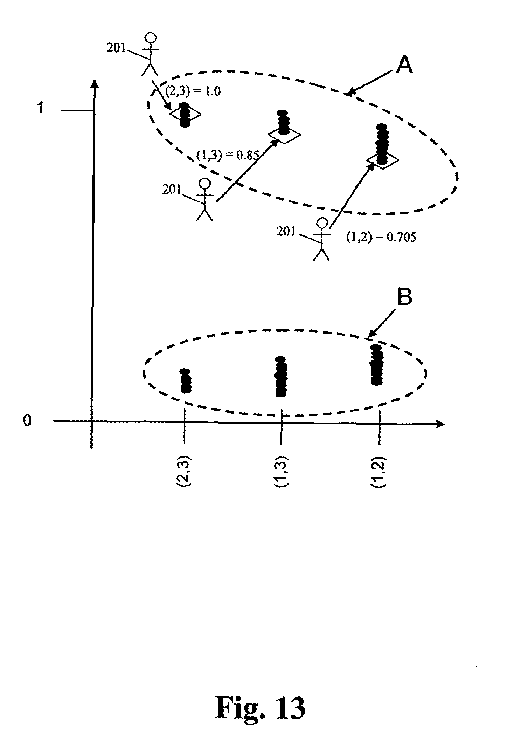

For two groups of reference subjects, group A and group B, we assume that $f = \{1,2,3\}$ is the set of features that are used and $N = 2$ is the combination parameter that determines the number of features to be combined (e.g. two features can be combined or three features, etc). The set of all non-repetitive combinations of features will be: $V = \{(1,2), (1,3),(2,3)\}$, i.e. the first element is a combination of features 1 and 2, the second is a combination of features 1 and 3, etc. Based on the above, (1,2) is a first property, (1,3) is a second property and (2,3) is a third property. Figs. 10-12 illustrate schematically a possible distribution for these properties for all the reference subjects in groups A and B. Fig. 10 shows the statistical distribution of the property (1,2) for the two groups A and B where the reference subjects In the groups are plotted In accordance to the ("1","2") feature values (i.e. "1" is the feature 1 value and "2" is the feature 2 value). The domain A shows the distribution of the reference subjects (marked with circles) in group A, and domain B shows the distribution for the reference subjects in group B (marked with squares). Figs. 11 and 12 show the corresponding statistical distributions of properties (1,3) and (2,3), respectively, i.e. for the (1,3) distribution of properties as shown in Fig. 3 all the ("1","3") feature values for all the reference subjects are plotted and for the (2,3) statistical distribution in Fig. 13 all the ("2","3") feature values for all the reference subjects are plotted.

Continuing with the example, the subsequent step is to calculate the posterior probability vector for each respective person in groups A and B. For clarification, an example of feature values ("1","2"), ("1","3") and ("2","3") for subject 201 that is classified in group A is shown in Figs. 10-12. The posterior probability vector for this particular subject is determined by calculating the probability that the subject 201 belongs to group A. For this particular subject it is clear that the subject lies within domain A and not at the boundaries or even within domain B which results in a probability vector having elements of high probability for each respective property, i.e. the variance of the vector is high. The result of the probability vector could accordingly be P=[0.79;0.85;1.0], i.e. the probability the subject 201 lies within domain A for properties (1,2), (1,3) and (2,3) is 0.79, 0.85 and 1.0, respectively. As mentioned previously, the probability vector is calculated for all the subjects in domains A and B. For the subjects in group 8, the resulting probability vectors will have low values since group B is used as a reference group. Accordingly, a probability vector P=[0.09;0.05;0.0] for a subject in group B Indicates that the probability that the subject B is within group B is (very) large, i.e. the variance is large

Furthermore, after the calculation an evaluation process is performed to evaluate which probability vectors are to be used, i.e. which probability vectors have sufficiently large variance. This "filtering process" is necessary for separating the two groups from each other as discussed previously in Figs. 5-7. As an example, instead of values of 0.705, 0.85 and 1.0, the result for another subject from group A could be 0.5, 0.49 and 1.0 (this could be a person that lies within the overlapped domain). In this case the probability vector might not be used due to the low variance, or the first two elements could be neglected. For evaluating the probability vectors a threshold value may be defined whereby all probability vectors or the elements within the vectors having a variance below the pre-defined threshold value may not be used. As an example, for a threshold value of 0.6 it might be sufficient that only one element in the probability vector is below 0.6 in order not to use the probability vector.

Figure 13 shows the resulting reference tool after filtering out the vectors or vector elements that do not fulfill the filtering criterion (i.e. the threshold value) so that only those probability vectors that have large variance for group A and B subjects are selected. This corresponds to the reference tool shown in Fig. 7, but here the number of groups is only two. The number could of course be more than two, e.g. three, as shown in Fig. 7, or more than three.

In this graph the selected probability vectors are plotted for all the reference subjects, where the x-axis stands for the property elements (1,2), (1,3), (2,3), and the y-axis for the associated probability values. For clarification, the probability vector P=[0.705;0.85;1.0] for subject 201 from group A is shown for the three property elements along the resulting probability vectors for the other reference subjects from group A (marked with filled circles). Fig. 13 shows the corresponding result for the subjects from group B. It should be obvious from the example that since group B was selected as a reference group when calculating the probability vectors, all the reference subjects from group A are in the upper part and the reference subjects B are in the lower part.

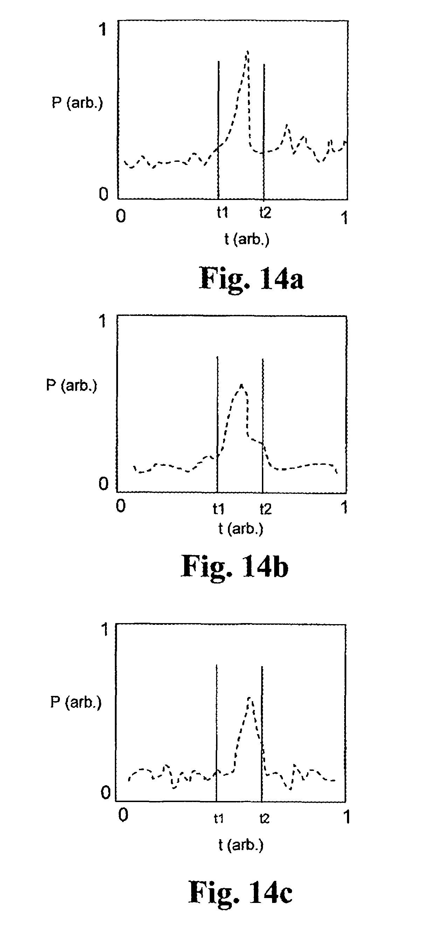

Figure 14a-c illustrates graphically an example of how to define features from biosignal data, e.g. EEG data. Imaginary biosignal data for three reference subjects (the number of reference subjects is of course much larger) within the same group is shown. The vertical axis is e.g. the power of the spontaneous potentials P for said electrode and the horizontal axis may be the time t. The units on the axis could e.g. be arbitrary units (arb.). As shown here, there is a clear correlation between the three peaks considering the common peaks in the time window between t1 and t2. Accordingly, the intensity of the peak, or simply the presence of a peak within this time window could be used to define a feature for these reference subjects (assuming that other reference subjects show a similar characteristic).

Preferably, said reference features are selected based on selection criteria, including said selection criteria of high correlation between the reference persons. Accordingly, prior to selecting which reference features are to be used, a correlation check is performed for each reference person and based on the correlation check the preferred reference features are selected. This correlation check could e.g. comprise scanning all the peaks in the diagram in Fig. 14a-c and detecting which of the peaks are maximum peaks, then detecting where said maximum peaks are located, i.e. whether they are within a same time window. Another check would also be to calculate the spectral entropy for all the reference persons and compare the resulting entropy, i.e. whether there is large deviation in the resulting value or not. Accordingly, a standard correlation check could be performed by e.g. determining the absolute delta power, the absolute theta power, the absolute alpha power, the absolute beta power and the absolute gamma power. A further correlation check could Include determining the relative delta power, the relative theta power, the relative alpha power, the relative beta power, and the relative gamma power. A still further check could include determining the total power, the peak frequency, the median frequency, the DFA scaling exponent (alpha band oscillations), and the DFA scaling exponent (beta band oscillations).

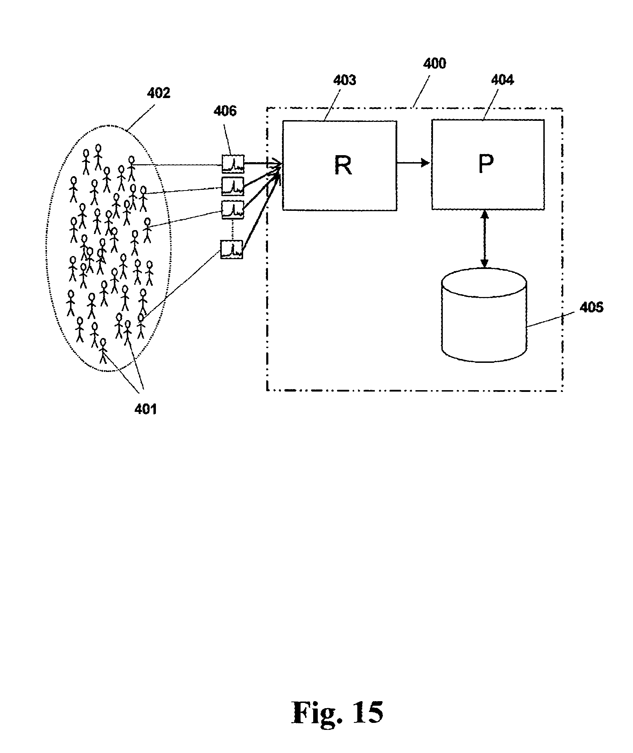

Figure 15 shows an apparatus 400 for constructing said reference tool based on biosignal data 406 collected from one or more groups 402 of reference subjects 401. The apparatus 400 comprises a receiver (R) 403, a processor (P) 404 and a memory 405. The receiver (R) 403 is adapted to receive biosignal data 406 from the reference subjects 401 in the reference group 402. The processor is adapted to perform the method steps shown in Fig. 9 based on instructions in e.g. software stored in the memory 405 which may be a programmable memory. This includes performing said pre-scan to check whether there is a correlation in the received biosignal data 406 from the reference persons 401. Thereafter the processor (P) 404 extracts/defines said reference features, and subsequently calculates said posterior probability vectors for all the reference subjects and filters out those vectors or vector elements that are not in accordance with said threshold value. The remaining vectors or vector elements are then implemented for constructing said reference distribution for said reference subjects as a function of said domain elements. The reference distribution is then stored in the memory 405.

Depending on the type of biosignal measurement, the processor (P) 404 is further adapted to convert the biosignal measurements 406 into biosignal data. This might be the case in MRI measurements where the resulting images need to be converted into biological data, e.g. light intensity.

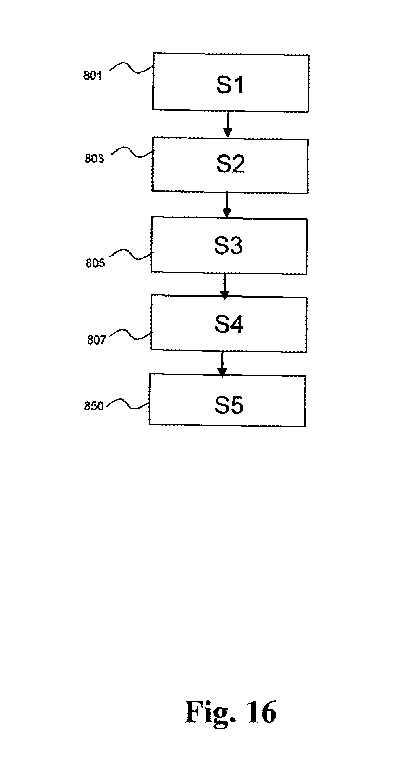

Figure 16 shows a flow diagram of method steps for using said reference tool 21 to diagnose a subject, wherein initially a biosignal measurement is performed (S1) 801 on the subject. As mentioned previously, the biosignal measurement can e.g. comprise EEG measurement, magnetic resonance imaging (MRI), functional magnetic resonance imaging (FMRI), magneto-encephalographic (MEG) measurements, positron emission tomography (PET), CAT scanning (computed axial tomography), single photon emission computerized tomography (SPECT) and the like.

Eventually, the information resulting from said biosignal measurements must be converted to biosignal data (S2) 803, e.g. if the biosignal data comprise imaging data where the image is preferably converted to digital data, e.g. based on the light intensity or other similar properties.

Then, the features corresponding to said reference features are extracted from the biosignal data (S3) 805. This can comprise said intensity peak within said time window t1 and t2 as an example shown in Fig. 6. Accordingly, the processor which will be discussed in more detail below is pre-programmed in a way so that it extracts the features from the biosignal data (S2) 803 which correspond to the pre-stored reference features.

The posterior probability vector as defined in the reference tool is now calculated for the subject (54) 807. Considering Fig. 13, the posterior probability vector that is calculated from the reference features is P=[(2,3),(1,3),(1,2)] and compared to the distribution shown e.g. in Fig. 5 and based thereon the subject is classified. The result thereof is that it is possible to see whether the subject falls within the distribution for groups A or B or not (S5) 809. As an example, assuming that group A is a well defined group and that all the elements in the probability vector for the subject fall within the feature distribution in Fig. 5, this would clearly indicate that the subject belongs to group A. This means that if all the subjects in group A are healthy subjects, this could indicate that the subject is a healthy subject. However, if the subject does no fall within group A that obviously means that the subject does not belong to this particular group. However, if the subject belongs partly to group A, further processing Is needed. In this example, it might be preferred that e.g. the age and the gender of the reference subjects In group A match the age and the gender of the test subjects.

The above mentioned method steps can be implemented in software or hardware.

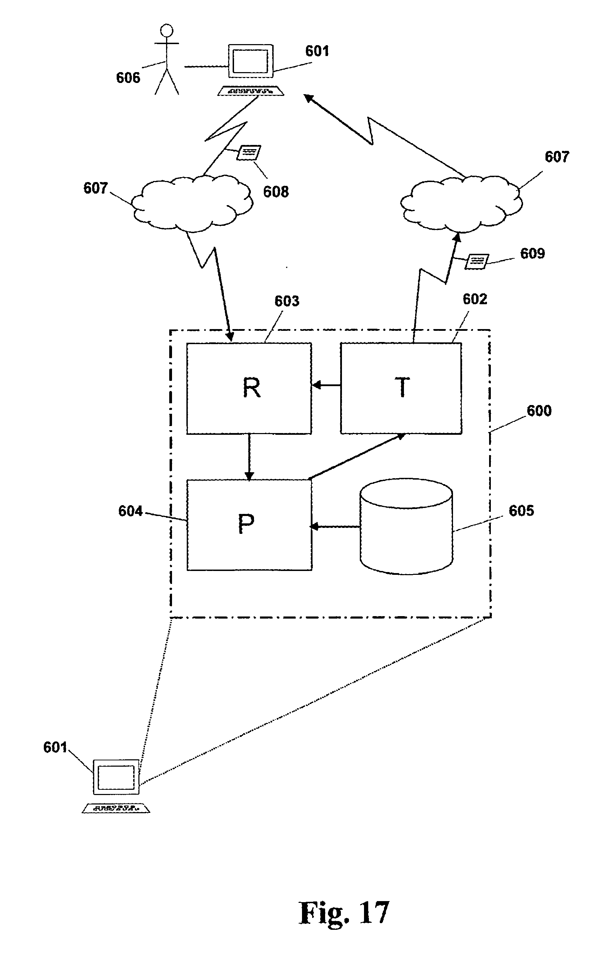

Figure 17 shows an apparatus 600 according to the present invention for using said pre-stored reference tool for generating a discriminatory signal for indicating a medical condition of a subject based on biosignal data collected from the subject. In this embodiment the apparatus 600 comprises a receiver (R) 603, a transmitter (T) 602, a processor (P) 604 and a memory 605 which is preferably a programmable memory.

In one embodiment the apparatus 600 is implemented as a service system, wherein the receiver 603 is adapted to receive biosignal data 608 from a subject measured by e.g. a doctor 606 or a technician. These data, which can e.g. be said EEG data, are then transmitted over a communication channel 607, e.g. the Internet, directly from the measuring device or a computer 601 interconnected to the measuring device. The biosignal data 608 received by said receiver (R) 603 are then processed by the processor (P) 604., which calculates the features for the subject and the posterior probability vector and compares the probability vector with the features distribution (e.g. as illustrated in Fig. 7) in order to check whether the subject belongs to a certain reference group or groups that are well defined.

In one embodiment the result in the comparison could be an issue of a "report" 609 telling whether the test person is diagnosed as positive/negative diagnosed. This report is then transmitted by the transmitter (T) 602 via said communication channel 607 back to the doctor 606 or the technician.

In another embodiment, said receiver (R) 603, transmitter (T) 602, processor (P) 604 and memory 605 can be standard hardware components in a computer system comprised in said apparatus. The apparatus can comprise an EEG measuring device or any other kind of device depending on the application. These hardware components can also be hardware components in the computer 601, wherein the memory has pre-stored software adapted to instruct the processor to perform the method steps in Fig. 16.

Begin EXAMPLE 2

The following example illustrates the importance of using said reference tool in a clinical trial. The participants in the trial were divided in two groups. One group consisted of 10 elderly subjects that have been diagnosed with mild to moderate dementia of the Alzheimer's type (AD group). A second age-matched group of 10 healthy individuals (i.e. non-AD individuals) was included as a control group.

The AD-group of participants consisted of patients in a follow-up surveillance in the memory clinic at the Department of Geriatrics in Landspitall University Hospital, Reykjavik, Iceland. The group consisted of patients with Alzheimer's disease (AD) according to ICD-10. The other group consisted of normal Control participants who were recruited among relatives of demented patients attending a day-care center.

To be eligible for participation in the study the subjects had to be in between 60-80 years of age, in good general health as determined by standard physical examination, and with no acute changes on ECG. Exclusion criteria included smoking or any other use of tobacco (also excluding those that had stopped tobacco use about a week or less prior to the trial), treatment with neuroleptics and benzodiazepines, impaired liver- or kidney function, hypersensitivity to scopolamine, indication of drug, alcohol or medical abuse, glaucoma or possibility of raised intraocular pressure with administration of scopolamine. Prior to the screening visit the subjects were interviewed by phone. The AD subjects were selected from hospital records. All the AD patients in the follow-up program at the Memory Clinic were being treated with anti-dementia drugs. To minimize the variation between the subjects In the trial, the participants In the AD-group were selected from patients that were being treated with the same cholinesterase-inhibitor, Reminyl® (galantamine HBr).

In the screening visit the participants underwent physical examination by the study physician and fulfillment of the inclusion/exclusion criteria set forth. Information of diagnosis, ECG recording, blood sampling, staging (Global Deterioration Scale (GDS) and MMSE (see Table 1) and CT/SPECT were recorded, and finally an examination was carried out by an ophthalmologist.

Electroencephalographic neurophysiological signals were recorded from each of the subjects. The recording protocol was divided into two parts or sessions which were identical.

In this example, at least one probe compound was used to initiate a neurophysiologic effect. As mentioned above, the aim of this example is to illustrate the importance of the reference tool for diagnosing subjects, which is just as well presented whether or not the compound is used or not. In some cases, the use of such a compound is necessary to initiate a neurophysiological effect, and in some cases the use of such compound is not necessary. The probe compound is adapted to initiate a neurological reaction when administered to a subject, wherein the selection of the compound must be such that the neurological reaction caused for a subject suffering from a particular neurological disease is different than that caused for a healthy subject. All the subject were administered the same dose of the probe compound and the time interval between the administration and the onset of post-administration measurements was substantially Identical for all measured subjects.

The compound used in this particular example was scopolamine, which was administered intravenously. Within each section a two-minute period was recorded while the subject was instructed to be at rest with eyes closed. The data collected from these periods were used to estimate the individual features. The substance scopolamine was chosen based on its effects perturbing biophysical pathways that are known to deteriorate In subjects suffering from Alzheimer's disease. Scopolamine is a cholinergic antagonist, and it is well known that the cholinergic system deteriorates in Alzheimer's disease patients.

Table 1. Characteristics of the participants examined in the study.

|  | Number | Male | Female | Average Age | GDS# | MMSE |
|---|---|---|---|---|---|---|
| Controls | 10 | 3 | 7 | 72.6 SD 5.3 | 1.2 SD 0.4 | 29.1/30 SD 0.9 |
| AD | 10 | 7 | 3 | 75.9 SD 3.0 | 4.3 SD 0.5 | 21.2/30 SD 2.6 |

#Global Deterioration Scale (GDS) for age-associated cognitive decline and Alzheimer's disease: stage 1: No cognitive decline (normal), stage 2: very mild cognitive decline (forgetfulness); stage 3: mild cognitive decline (early confusional); stage 4: moderate cognitive decline (late confusional); stage 5: Moderately severe decline (early dementia); Stage 6: severe cognitive decline and stage 7: very severe cognitive decline. The last two did not participate in this study. SD indicates one standard deviation from the mean.

The electroencephalographic signals were recorded using computerized measuring equipment. The recordings were performed using the conventional International 10-20 system of electrode placement. The collected data were stored in raw format on a storage device for later analysis. During the recordings the signals are displayed simultaneously on a computer screen for allowing the operator to monitor if electrodes come loose and to enter marks that indicate specific events. Such events may indicate initiation of specific parts of the recording protocol or occurrences that may lead to artifacts being present in the recordings. Such occurrences include that the subject blinks his eyes, swallows, moves or in general breaches protocol.

When all the data had been collected features that characterize the individual recordings were extracted. Identical features were extracted from the first and second recording sessions of the protocol. Features extracted were derived from results reported in the scientific literature (Adler G. et al. 2003, Babiloni C. et al. 2004, Bennys K. et al. 2001, Brunovsky M. et al. 2003, Cichocki et al. 2004, Cho S.Y. 2003, Claus J.J. et al. 1999, Hara J. et al. 1999, Holschneider D.P. et al. 2000, Hongzhi Q.I. et al. 2004, Huang C. et al. 2000, Hyung-Rae K. et al. 1999, Jelles B. et al. 1999, Jeong J. et al. 1998, 2001, 2004, Jonkman E.J. 1997, Kikuchi M. et al. 2002, Koenig T. et al. 2004, Locatelli T. et al. 1998, Londos E. et al. 2003, Montplaisir J. et al. 1998, Moretti et al. 2004, Musha T. et al. 2002, Pijnenburg Y.A.L. et al. 2004, Pucci E. et al. 1998,1999, Rodriquez G. et al. 1999, Signorino M. et al. 1995, Stam C.J. et al. 2003, 2004, Stevens A. et al. 1998, 2001, Strik W.K. et al. 1997, Vesna J. et al. 2000, Wada Y. et al. 1998, Benvenuto J. et al. 2002, Jimenez-Escrig A. et al. 2001, Sumi N. et al. 2000), which are hereby incorporated by reference.

The features used in the example were numbered as follows. 16 base features were selected.

1. Absolute delta power
2. Absolute theta power
3. Absolute alpha power
4. Absolute beta power
5. Absolute gamma power
6. Relative delta power
7. Relative theta power
8. Relative alpha power
9. Relative beta power
10. Relative gamma power
11. Total power
12. Peak frequency
13. Median frequency
14. Spectral entropy
15. DFA scaling exponent (alpha band oscillations)
16. DFA scaling exponent (beta band oscillations).

These features were evaluated using a part of the first section where the subjects were at rest with eyes closed. The same features were estimated for the corresponding second section which occurs after administration of the scopolamine. These features post-administration are enumerated 17-32. Finally the features are combined in order to obtain a measure of the response of each feature to the administration of the scopolamine, by determining the ratio of the same feature before and after drug administration. These combination features are enumerated 33-48. (For example feature 33 is the ratio of feature 1 and 17.) Many other combinations of the features before and after administration will reflect the response as well, e.g. the difference.

In order to demonstrate the effectiveness of using a probe compound the following analysis was performed. The features were used to classify the two groups using a pattern classification scheme.

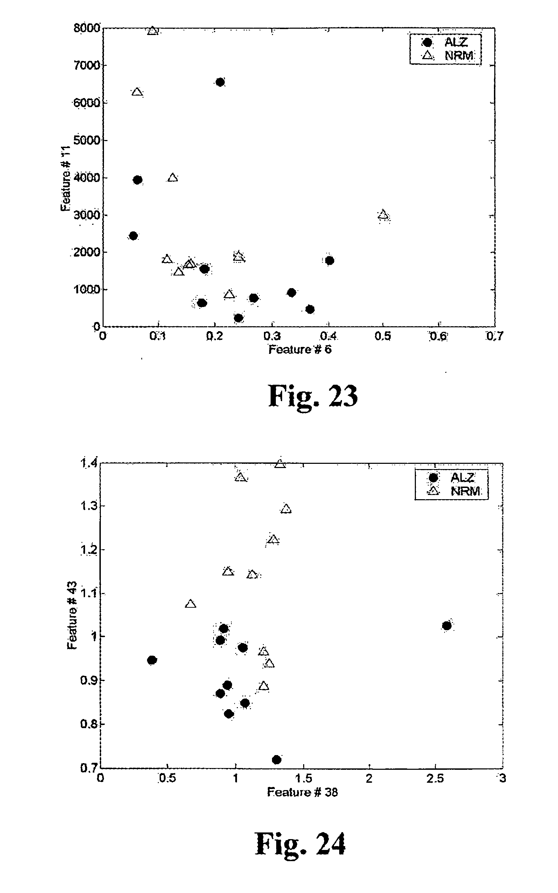

In order to design a classifier a labeled training set is required (supervised learning). The classifier is then used to classify unseen data. In order to evaluate the performance of a classifier an independent test set is required. The classifier is a multidimensional pattern recognition method e.g. k-NN nearest neighbor scheme (k-NN), support vector machines (SVM), linear discriminant analysis, quadratic discriminant analysis, regularized discriminant analysis, Logistic regression, naive Bayes classifier, hidden Markov model classifiers, neural network based classifiers including multi-layer perceptron networks and radial basis function networks, support vector machines (SVM), Least-squares SVM, Classification Tree-based classifiers including Classification and Regression Trees (CART), ID3, C4.5, C5,0, AdaBoost, and ArcX4, Tree-based ensemble classifier including Random Forests™. The chosen classifier is then used to construct a boundary in feature space that best separates the groups according to criteria the particular classifier is based on. From the found boundary the posterior probability is estimated, usually as function of distance from the boundary and from the estimated distribution of the training set, e.g. the data from the groups, parameterized as a function of the distance from the said boundary. A training dataset with two groups (10 in each) cannot support classification taking into account more than 2 features at the time without risk of running into over fitting problems. Over fitting leads to classifiers that in general perform poorly on unseen data. In the present example two features at a time were considered. Figure 23 and 24 illustrate the effect of the scopolamine. In Figure 23 the features stem from measurements before administration of the scopolamine, while Figure 24 demonstrates the response of the same features by considering the ratio of their values pre- and post-administration. Evidently the scopolamine leads to significantly better separation between the groups for this feature pair.

All possible combinations of features were considered. Hence, if d features are taken into account $d(d+1)/2$ possible pairs were tested. For each pair the classification performance or accuracy was estimated by applying the "leave one out" scheme as follows. Let N be the total number of elements in the training set. The scheme is based on constructing N new training sets each with N-1 elements, where each element of the initial training set is left out once. For each resulting training set the element left out constitutes the test set. The overall performance is estimated with ratio of incorrect classifications of test sets to N.

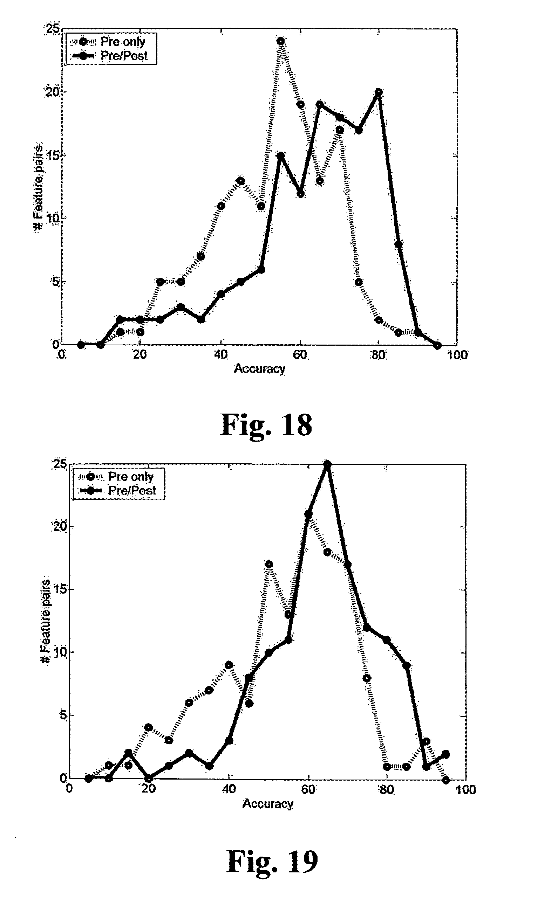

The efficiency of applying a pattern enhancing substance, scopolamine in this example, was demonstrated by considering the histogram of classification performance for two distinct feature sets, the set that only involves features extracted prior to substance administration, features 1-16, and the set that is sensitive to the response to the pattern enhancing substance, features 33-48, i.e., the ratios of the basic features before and after administration. The sets are of equal size. The features were estimated from the P3-P4 montage. Figure 18 illustrates the comparison between the classification performances for the two sets evaluated using the 3-NN scheme. As is evident for this example, the pattern enhancing substance leads to substantially enhanced classification performance. The number of feature pair classifiers scoring 80% or better goes from 4 to 29. Figure 19 illustrates the same comparison, but using the SVM classification scheme to obtain the feature pair classifiers. The number of feature pair classifiers scoring 80% or better goes from 5 to 23. This demonstrates that using a probe compound leads to a signal that is more discriminatory.

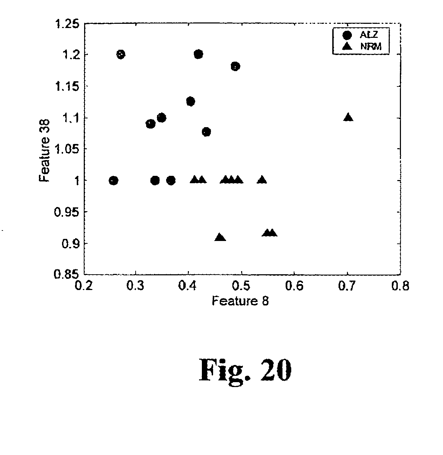

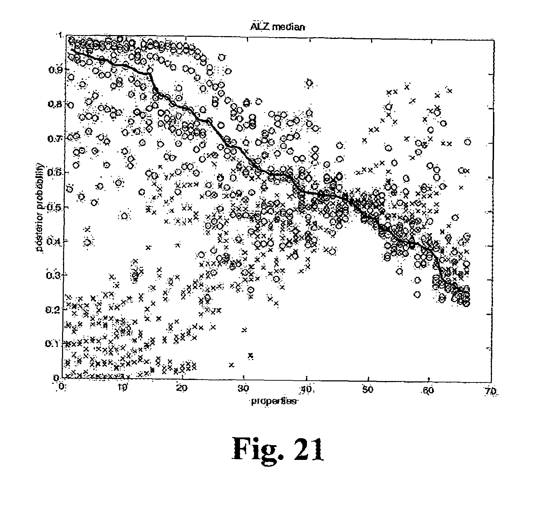

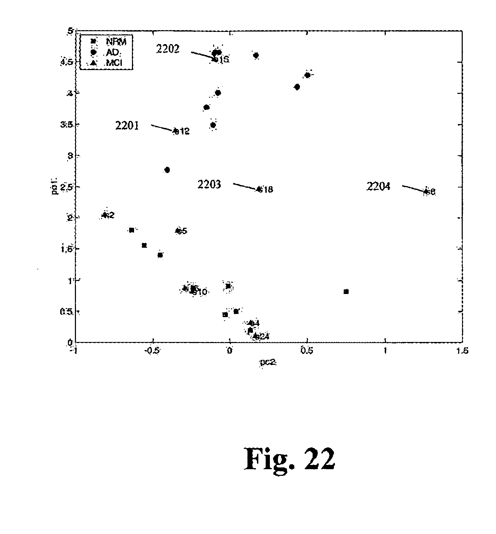

Next we demonstrate how the database is constructed. Again working with two features in each property figure 20 shows the distribution for one property. For each property the posterior probability is calculated using a SVM classifier. Figure 21 illustrates the posterior probabilities for two subjects, an Alzheimer's subject, circles, and a control subject, crosses. The solid line illustrates the median for the whole Alzheimer's group. It is practical in order to minimize destructive interference to include only properties with median posterior probability above some chosen threshold, e.g. 0.8. Figure 22 illustrates the distribution for the Alzheimer's group and the control group in terms of the pca-posterior probabilities.

In order to demonstrate the predictability, e.g. the diagnostic value, of the invention a third group was Included in the clinical trial described above. This group was recruited from subjects that have been classified as having mild cognitive impairment (MCI). The group was age matched to the other groups. It is well known that about 12% of MCI subjects will receive diagnosis as Alzheimer's patients within one year. In Figure 22 the results for this group are illustrated following the classification procedure outlined above, e.g. each subject is compared to the database constructed from the data from the first two groups. The invention predicted that subject s12 2201 and s16 2202 belong to the Alzheimer's group. The group was followed up one year after the clinical trial was conducted. It turned out that two subjects were diagnosed with dementia of the Alzheimer's type, the same subjects that the invention predicted belonged to that group one year prior to the follow up visit. This demonstrates that the invention is capable of detecting individuals with neurological condition dementia of the Alzheimer's type one year earlier than a conventional diagnosis workup is capable of. In other words the invention is capable of early diagnosis of Alzheimer's disease. Subjects s18 2203 and s6 2204 were predicted to belong to neither the Alzheimer's group nor the control group. Two years later these subjects were followed up for a standard workup, one was then known to have suffered a stroke while the condition of the other was uncertain. It was however clear that cognitive impairment was beyond doubt. It is speculated that these subjects have the vascular dementia or micro vascular dementia and should therefore not be classified with either group in the database in accordance with the result obtained from the invention.

The above mentioned compound may comprise one or more compounds from the group of consisting of GABA affecting drugs including propofol and etomidate; barbiturates including methohexital, thiopental, thiamylal, buthalital, thialbarbital, hexobarbital, pentobarbital, secobarbital, hexethal, butalbital, cyclobarbital, talbutal, phenobarbital, mephobarbital, and barbital; benzodiazepines such as alprazolam, bromazepam, chlordiazepoxide, clobazam, clonazepam, clorazepate, clozapine, olanazapine diazepam, estazolam, flunitrazepam, flurazepam, halazepam, ketazolam, loprazolam, lorazepam, lormetazepam, medazepam, midazolam, nitrazepam, nordazepam, oxazepam, prazepam, ouazepam, temazepam, and triazolam; cholinergic agonists such as aceclidine, AF-30, AF150, AF267B, alvameline, arecoline, bethanechol, CDD-0102, CDD-0034-C, CDD-0097-A, cevimeline, CI 1017, cis-dioxolane, milameline, muscarine, oxotremorine, pilocarpine, RS86, RU 35963, RU 47213, sabcomeline, SDZ-210-086, SR 46559A, talsaclidine, tazomeline, UH5, xanomeline, and YM 796; cholinergic antagonists including AF-DX 116, anisotropine, aprophen, AQ-RA 741, atropin, belladonna, benactyzine, benztropine, BIBN 99, DIBD, cisapride, clidinium, darifenacin, dicyclomine, glycopyrrolate, homatropine, atropine, hyoscyamine, ipratropium, mepenzolate, methantheline, methscopolamine, PG-9, pirenzepine, propantheline, SCH-57790; SCH-72788, SCH-217443, scopolamine, tiotropium, tolterodine, and trihexyphenidyl; acetyl choline esterase (ACE) inhibitors including 4-aminopyridine, 7-methoxytacrine, amiridine, besipirdine, CHF2819, CI-1002, DMP 543, donepezil, eptastigmine, galantamine, huperzine A, huprine X, huprine Y, MDL 73745, metrifonate, P10358, P11012, phenserine, physostigmine, ouilostigmine, rivastigmine, Ro 46-5934, SM-10888, suronacrine, T-82, tacrine, TAK-147, tolserine, trifluoroacetophenone, TV3326, velnacrine, and zifrosilone; ACh release enhancers including linopirdine, and XE991; Choline uptake enhancers including MKC-231, and Z-4105; nicotinic agonists including: A8T-089, ABT-418, GTS-21, and SIB-1553A; NMDA antagonist including ketamine, and memantine; serotonin inhibitor such as cinanserin hydrochloride, fenclonine, fonazine mesylate, and xylamidine tosylate; serotonin antagonist including altanserin tartrate, aAmesergide, cyproheptadiene, granisetron, homochlorcyclizine, ketanserin, mescaline, mianserin, mirtazapine, perlapine, pizotyline, olanzapine, ondansetron, oxetorone, risperidone, ritanserin, tropanserin hydrochloride, and zatosetron; serotonin agonists including 2-methylserotonin, 8-hydroxy-DPAT, buspirone, gepirone, Ipsapirone, rizatriptan, sumatriptan, and zolmatriptan; serotonin reuptake inhibitors including citalopram, escitalopram oxalate, fluoxetine, fluvoxamine, paroxetine, and sertraline; dopamine antagonists including pimozide, ouetiapine, metoclopramide, and dopamine precursors including levodopa, as well as other compounds that affect the brain and nerve system.

As appears from the description herein, the present invention is particularly suited for using biosignal data obtained by electroencephalographic (EEG) measurements, in the absence or the presence of the one or more of the compounds. However, other multidimensional biosignal measurement techniques used in neurophysiologic studies may as well be used in the methods of the invention, separately or in a combination of one or more techniques. Such techniques include without limitation magnetic resonance imaging (MRI), functional magnetic resonance imaging (FMRI), magnetoencephalographic (MEG) measurements, positron emission tomography (PET), CAT scanning (Computed Axial Tomography) and single photon emission computerized tomography (SPECT).

When referring to one biosignal measurement' in the context herein, what is meant is a measurement of biosignal data with one technique such as any of the above, i.e. one biosignal measurement' used in the method herein will give rise to multidimensional data. In all cases it is implied that the data is computerized, e.g. images digitized and so forth. End Example

Certain specific details of the disclosed embodiment are set forth for purposes of explanation rather than limitation, so as to provide a clear and thorough understanding of the present invention. However, it should be understood by those skilled in this art that the present invention may be practiced in other embodiments that do not conform exactly with the details set forth herein, without departing significantly from the spirit and scope of this disclosure. Furthermore, in this context, and for the purposes of brevity and clarity, detailed descriptions of well-known apparatuses, circuits and methodologies have been omitted so as to avoid unnecessary detail and possible confusion.

Reference signs are included in the claims. However, the inclusion of the reference signs is only for clarity reasons and should not be construed as limiting the scope of the claims.

**Claims**

1. A method of constructing a reference tool (21) based on biosignal data (7, 8, 406) collected from one or more groups (1, 2, 402) of reference subjects (4, 5, 401), wherein each group represents reference subjects having at least one common characteristic, the method comprising:

   • defining one or more reference features (11, 12) representing a common characteristic of the reference subjects within the same group (1, 2, 402),
   • determining (105) reference feature values for said reference features (10, 11, 12) for each respective reference subject (4, 5, 401),
   • defining a feature property domain (13) comprising domain elements (14-16) where each element is defined by one of said reference features (11, 12) or a combination of two or more of said reference features (11, 12),
   • determining (107) a posterior probability vector (17, 17-17c) for each respective reference subject (4, 5, 401) based on said feature property domain (13), wherein each respective element (18-20) of the posterior probability vector (17) refers to said domain elements (14-16) and indicates the probability (18-20) that a reference subject (4, 5, 401) of a particular group (1, 2, 406) belongs to said group in terms of said domain elements (14-16),
   • applying (109) a filtering process on said posterior probability vectors (17, 17-17c), said filtering process being based on removing those vectors (17c) or vector elements that are above or below a pre-defined threshold value, the remaining vectors or vector elements being implemented for constructing a reference distribution (46) for said reference subjects (4, 6, 401) as a function of said domain elements (14-16).

2. A method according to claim 1, wherein the reference features (10, 11, 12) are selected from a group consisting of the absolute delta power, the absolute theta power, the absolute alpha power, the absolute beta power, the absolute gamma power, the relative delta power, the relative theta power, the relative alpha power, the relative beta power, the relative gamma power, the total power, the peak frequency, the median frequency, the spectral entropy, the DFA scaling exponent (alpha band oscillations), the DFA scaling exponent (beta band oscillations) and the total entropy.

3. A method according to claim 1 or 2, wherein the domain elements (14-16) of said feature property domain comprise non-repetitive combinations of two or more of said reference features.

4. A method according to any of the preceding claims, wherein the step of determining (107) the posterior probability vector is based on a calculation method selected from a group consisting of: k-NN nearest neighbor scheme (k-NN), support vector machines (SVM), linear discriminant analysis, quadratic discriminant analysis, regularized discriminant analysis, Logistic regression, naive Bayes classifier, hidden Markov model classifiers, neural network based classifiers including multi-layer perceptron networks and radial basis function networks, support vector machines (SVM), Least-squares SVM, Classification Tree-based classifiers including Classification and Regression Trees (CART), ID3, C4.5, C5.0, AdaBoost, and ArcX4, Tree-based ensemble classifier including Random Forests™.

5. A method according to any of the preceding claims, wherein the biosignal data (7, 8, 406) within the same groups (1, 2, 402) is collected during similar activity of the reference subjects (4, 5, 401) within the same group.

6. A method according to any of the preceding claims, wherein said characteristics of the reference subjects (4, 5, 401) are selected from the following characteristics:

• the gender of the subject,
• the habit-related actions performed by the subjects,
• the medical condition of the subjects,
• the particular neurological disease of the subjects,
• the specific handedness of the subjects,
• the age of the subjects,
• whether the subject is healthy or not,
• the physical condition of the subjects.

7. A computer program product for instructing a processing unit to execute the method step of any of the claims 1-6 when the product is run on a computer.

8. An apparatus (400) for constructing a reference tool (21) based on biosignal data (7, 8, 406) collected from one or more groups (1, 2) of reference subjects (4, 5), wherein each group represents reference subjects having at least one common characteristic, the apparatus comprising:

• a processor (404) for pre-scanning the biosignal data and based thereon defining one or more reference features (11, 12) representing a common characteristics of the references subjects within the same group (1, 2, 402),
• a processor (404) for determining (105) reference feature values for said reference features (10, 11, 12) for each respective reference subject (4, 5, 401),
• means for defining a feature property domain (13) comprising domain elements (14-16) where each element is defined by one of said reference features (11, 12) or a combination of two or more of said reference features (11, 12),
• a processor (404) for determining (107) a posterior probability vector (17, 17-17c) for each respective reference subject (4, 5, 401) based on said feature property domain (13), wherein each respective element (18-20) of the posterior probability vector (17) refers to said domain elements (14-16) and indicates the probability (18-20) that a reference subject (4, 5, 401) of a particular group (1, 2, 406) belongs to said group in terms of said domain elements (14-16),
• a processor (404) for applying (109) a filtering process on said posterior probability vectors (17, 17-17c), said filtering process being based on removing those vectors (17c) or vector elements that are above or below a pre-defined threshold value, the remaining vectors or vector elements being implemented for constructing a reference distribution (46) for said reference subjects (4, 6, 401) as a function of said domain elements (14-16).

9. An apparatus according to claim 8, further comprising a receiver (403) adapted to receive biosignal data (7, 8, 406) from said reference subjects (4, 5, 401).

10. A method of using a pre-stored reference tool (21) for generating a discriminatory signal for indicating a medical condition of a subject (50, 606) based on biosignal data (51, 608) collected (801) from the subject (50, 606), the reference tool (21) being constructed based on biosignal data collected from one or more groups (1, 2, 502) of reference subjects (4, 5, 401), each group (1, 2, 402) representing reference subjects (4, 5, 401) having at least one common characteristic, said reference tool being constructed by means of:

defining one or more reference features (11, 12) representing a common characteristic of the references subjects within the same group (1, 2, 402),
determining (105) reference feature values for said reference features (10, 11, 12) for each respective reference subject (4, S, 401),
defining a feature property domain (13) comprising domain elements (14-16) where each element is defined by one of said reference features (11, 12) or a combination of two or more of said reference features (11, 12),
determining (107) a posterior probability vector (17, 17-17c) for each respective reference subject (4, 5, 401) based on said feature property domain (13), wherein each respective element (18-20) of the posterior probability vector (17) refers to said domain elements (14-16) and indicates the probability (18-20) that a reference subject (4, 5, 401) of a particular group (1, 2, 406) belongs to said group in terms of said domain elements (14-16),
applying (109) a filtering process on said posterior probability vectors (17, 17-17c), said filtering process being based on removing those vectors (17c) or vector elements that are above or below a pre-defined threshold value, the remaining vectors or vector elements being implemented for constructing a reference distribution (46) for said reference subjects (4, 6, 401) as a function of said domain elements (14-16), wherein the method comprises:

      • determining (805) analogous feature values for the subject (50, 606),
      • determining (807) an analogous posterior probability vector for said subject,
      • evaluating (809) whether said posterior probability vector for said subject lies within said distribution for said reference subjects.

11. A method according to claim 10, wherein the features are selected from a group consisting of the absolute delta power, the absolute theta power, the absolute alpha power, the absolute beta power, the absolute gamma power, the relative delta power, the relative theta power, the relative alpha power, the relative beta power, the relative gamma power, the total power, the peak frequency, the median frequency, the spectral entropy, the DFA scaling exponent (alpha band oscillations), the DFA scaling exponent (beta band oscillations) and the total entropy.

12. A method according to claim 10 or 11, wherein said one or more biosignal data comprise electroencephalographic (EEG) data.

13. A method according to any of claims 10-12, wherein the biosignal data comprise data resulting from biosignal measurements selected from a group consisting of:

      • magnetic resonance imaging (MRI),
      • functional magnetic resonance imaging (FMRI),
      • magneto-encephalographic (MEG) measurements,
      • positron emission tomography (PET),
      • CAT scanning (Computed Axial Tomography) and
      • single photon emission computerized tomography (SPECT).

14. A method according to any of claims 10-13, wherein the medical condition is a neurological condition.

15. A method according to claim 6, wherein the neurological condition is selected from the group consisting of Alzheimer's disease, multiple sclerosis, mental conditions including depressive disorders, bipolar disorder and schizophrenic disorders, Parkinson's' disease, epilepsy, migraine, Vascular Dementia (VaD), Fronto-temporal dementia, Lewy bodies dementia; Creutzfeld-Jacob disease and vCJD ("mad cow's disease").

16. A method according to any of claims 10-15, wherein the groups (1, 2, 402) are defined such that they share at least one known characteristic of the subject (50, 606).

17. A computer program product for instructing a processing unit to execute the method step of any of claims 10-16 when the product is run on a computer.

18. An apparatus for using a pre-stored reference tool (21) for generating a discriminatory signal for indicating a medical condition of a subject (50, 606) based on biosignal data (51, 608) collected (801) from the subject (50, 606), the reference tool (21) being constructed based on biosignal data collected from one or more groups (1, 2, 502) of reference subjects (4, 5, 401), each group (1, 2, 402) representing reference subjects {4, 5, 401) having at least one common characteristic, said reference tool being constructed by means of:

      defining one or more reference features (11, 12) representing a common characteristic of the references subjects within the same group (1, 2, 402),
      determining (105) reference feature values for said reference features (10, 11, 12) for each respective reference subject (4, 5, 401),
      defining a feature property domain (13) comprising domain elements (14-16) where each element is defined by one of said reference features (11, 12) or a combination of two or more of said reference features (11, 12),
      determining (107) a posterior probability vector (17, 17-17c) for each respective reference subject (4, 5, 401) based on said feature property domain (13), wherein each respective element (18-20) of the posterior probability vector (17) refers to said domain elements (14-16) and indicates the probability (18-20) that a reference subject (4, 5, 401) of a particular group (1, 2, 406) belongs to said group in terms of said domain elements (14-16),
      applying (109) a filtering process on said posterior probability vectors (17, 17-17c), said filtering process being based on removing those vectors (17c) or vector elements that are above or below a pre-defined threshold value, the remaining vectors or vector elements being implemented for constructing a reference distribution (46) for said reference subjects (4, 6, 401) as a function of said domain elements (14-16), wherein the apparatus comprises:

• a processor (604) for determining analogous feature values for the subject (50, 606),
• a processor (604) for determining an analogous posterior probability vector for said subject,
• a processor (604) for evaluating whether said posterior probability vector for said subject lies within said distribution for said reference subjects.

19. An apparatus according to claim 18, further comprising a receiver (603) and a transmitter (602), wherein the receiver is adapted to receive said biosignal data (51, 608) from the subject (50, 606), and the transmitter is adapted to transmit the resulting generated discriminatory signal.

20. A use of a pre-stored reference tool (21) for generating a discriminatory signal for indicating a medical condition of a subject (50, 606) based on biosignal data (51, 608) collected (801) from the subject (50, 606), the reference tool (21) being constructed based on biosignal data collected from one or more groups (1, 2, 502) of reference subjects (4, 5, 401), each group (1, 2, 402) representing reference subjects (4, 5, 401) having at least one common characteristic, said reference tool being constructed by means of:

defining one or more reference features (11, 12) representing a common characteristic of the reference subjects within the same group (1, 2, 402),
determining (105) reference feature values for said reference features (10, 11, 12) for each respective reference subject (4, 5, 401),
defining a feature property domain (13) comprising domain elements (14-16) where each element is defined by one of said reference features (11, 12) or a combination of two or more of said reference features (11, 12),
determining (107) a posterior probability vector (17, 17-17c) for each respective reference subject (4, 5, 401) based on said feature property domain (13), wherein each respective element (18-20) of the posterior probability vector (17) refers to said domain elements (14-16) and indicates the probability (18-20) that a reference subject (4, 5, 401) of a particular group (1, 2, 406) belongs to said group in terms of said domain elements (14-16), applying (109) a filtering process on said posterior probability vectors (17, 17-17c), said filtering process being based on removing those vectors (17c) or vector elements that are above or below a pre-defined threshold value, the remaining vectors or vector elements being implemented for constructing a reference distribution (46) for said reference subjects (4, 6, 401) as a function of said domain elements (14-16), wherein the use comprises:

• determining (805) analogous feature values for the subject (50, 606),
• determining (807) an analogous posterior probability vector for said subject,
• evaluating (809) whether said posterior probability vector for said subject lies within said distribution for said reference subjects.

Fig. 1

Fig. 2

Fig. 3

Fig. 4

Fig. 5

Fig. 6

Fig. 7

**Fig. 8**

101    S1

103    S2

105    S3

107    S4

109    S5

**Fig. 9**

**Fig. 10**

**Fig. 11**

**Fig. 12**

**Fig. 13**

**Fig. 14a**

**Fig. 14b**

**Fig. 14c**

**Fig. 15**

Fig. 16

**Fig. 17**

## Fig. 18

## Fig. 19

**Fig. 20**

**Fig. 21**

**Fig. 22**

## Fig. 23

## Fig. 24

**European Patent Office**

**EUROPEAN SEARCH REPORT**

Application Number

EP 07 00 4338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CORDELLA L P ET AL: "Reliability parameters to improve combination strategies in multi-expert systems" PATTERN ANALYSIS AND APPLICATIONS, SPRINGER, NEW YORK, NY, US, vol. 2, no. 3, 1999, pages 205-214, XP002326666 ISSN: 1433-7541 * page 205 - page 209 * | 1-20 | INV. G06F19/00 |
| X | KITTLER J ET AL: "ON COMBINING CLASSIFIERS" IEEE TRANSACTIONS ON PATTERN ANALYSIS AND MACHINE INTELLIGENCE, IEEE SERVICE CENTER, LOS ALAMITOS, CA, US, vol. 20, no. 3, March 1998 (1998-03), pages 226-239, XP000767916 ISSN: 0162-8828 * page 226 - page 229 * | 1-20 | |
| X | LUDMILA KUNCHEVA: "TWO-LEVEL CLASSIFICATION SCHEMES IN MEDICAL DIAGNOSTICS" INTERNATIONAL JOURNAL OF BIO-MEDICAL COMPUTING, ELSEVIER SCIENCE PUBLISHERS, SHANNON, IE, vol. 32, no. 3 / 4, 1 May 1993 (1993-05-01), pages 197-210, XP000387194 ISSN: 0020-7101 * the whole document * | 1-20 | TECHNICAL FIELDS SEARCHED (IPC)<br><br>G06F |

-/--

The present search report has been drawn up for all claims

| Place of search | Date of completion of the search | Examiner |
|---|---|---|
| Munich | 9 July 2007 | SANANDRES LEDESMA, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

**EUROPEAN SEARCH REPORT**

Application Number

EP 07 00 4338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BAY S D: "Nearest neighbor classification from multiple feature subsets" INTELLIGENT DATA ANALYSIS ELSEVIER NETHERLANDS, vol. 3, no. 3, August 1999 (1999-08), pages 191-209, XP002416303 ISSN: 1088-467X * page 194, line 34 - page 198, line 22 * * page 203, line 19 - page 207, line 5 * ----- | 1-20 | |
| X | JEREBKO A K ET AL: "MULTIPLE NEURAL NETWORK CLASSIFICATION SCHEME FOR DETECTION OF COLONIC POLYPS IN CT CONOLOGRAPHY DATA SETS" ACADEMIC RADIOLOGY, RESTON, VA, US, vol. 10, no. 2, February 2003 (2003-02), pages 154-160, XP009049614 ISSN: 1076-6332 * page 480, left-hand column, line 6 - page 482, left-hand column, line 39 * ----- | 1-20 | |
| X | CHRISTODOULOU C I ET AL: "Medical diagnostic systems using ensembles of neural SOFM classifiers" ELECTRONICS, CIRCUITS AND SYSTEMS, 1999. PROCEEDINGS OF ICECS '99. THE 6TH IEEE INTERNATIONAL CONFERENCE ON PAFOS, CYPRUS 5-8 SEPT. 1999, PISCATAWAY, NJ, USA,IEEE, US, vol. 1, 5 September 1999 (1999-09-05), pages 121-124, XP010361455 ISBN: 0-7803-5682-9 * the whole document * ----- -/-- | 1-20 | TECHNICAL FIELDS SEARCHED (IPC) |

The present search report has been drawn up for all claims

| Place of search | Date of completion of the search | Examiner |
|---|---|---|
| Munich | 9 July 2007 | SANANDRES LEDESMA, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 07 00 4338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUDMUNDSSON STEINN ET AL: "Automatic sleep staging using support vector machines with posterior probability estimates" PROC. INT. CONF. COMPUTAT. INTELL. MODELL. CTRL. AUTOMAT. CIMCA 2005 INT. CONF. INTELL. AGENTS WEB TECHNOL. I-NET COMMERCE; PROCEEDINGS - INTERNATIONAL CONFERENCE ON COMPUTATIONAL INTELLIGENCE FOR MODELLING, CONTROL AND AUTOMATION, CIMCA 2005 AND INT, vol. 2, 2005, pages 366-371, XP002436132 * the whole document * | 1-20 | |
| A | GUDMUNDSSON S ET AL: "FC5:7 New diagnostic markers: Effects of scopolamine on EEG in Alzheimer?s disease" INTERNATIONAL PSYCHOGERIATRICS. AGING WITH DIGNITY. ABSTRACTS OF THE 12TH CONGRESS OF THE INTERNATIONAL PSYCHOGERIATRIC ASSOCIATION, vol. 17, no. Suppl-2, September 2005 (2005-09), pages 148-149, XP009066950 * the whole document * | 1-20 | TECHNICAL FIELDS SEARCHED (IPC) |

The present search report has been drawn up for all claims

| Place of search | Date of completion of the search | Examiner |
|---|---|---|
| Munich | 9 July 2007 | SANANDRES LEDESMA, J |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Multiple Neural Network Classification Scheme for Detection of Colonic Polyps in CT Conolography Data Sets. **JEREBKO A K et al.** Academic Radiology. February 2003, vol. 10, 154-160
- Medical diagnostic system using ensembles of neural SOFM classifiers. **CHRISTODOULOU C I et al.** Electronics, Circuits and Systems, 1999, Proceedings of ICECS '99 the 6th IEEE international conference. Pafos, 05 September 1999, vol. 1, 121-124
- An evaluation of ensembles methods in handwritten word recognition based on feature selection. **GUNTER S et al.** Pattern recognition, 2004, ICPR 2004. Proceedings of the 17th international conference. Cambridge, 23 August 2004, vol. 1, 388-392
- Statistical Pattern Recognition: A review. **JAIN A K et al.** IEEE Transaction on Pattern Analysis and Machine Intelligence. IEEE Service Center, January 2000, vol. 22, 4-37
- **DUDA et al.** Pattern Classification. John Wiley & Sons, Inc, 2001, 61
- Key Lab. of Complex Syst. & Intelligence Sci., Chinese Acad, of Sci., Beijing, China, Neural Networks. **QING TAO ; GAO-WEI WU ; FEI-YUE WANG ; JUE WANG.** IEEE Transactions on Publication Date. November 2005, vol. 16, 1561-1573